(19) Europäisches Patentamt
European Patent Office
Office européen des brevets

(11) Veröffentlichungsnummer: **0 199 675**
**A1**

# (12) EUROPÄISCHE PATENTANMELDUNG

(21) Anmeldenummer: **86810170.0**

(22) Anmeldetag: **11.04.86**

(51) Int. Cl.⁴: **C07D 499/00** , A61K 31/43 , //C07F9/65,C07F7/18

(30) Priorität: **17.04.85 CH 1635/85**

(43) Veröffentlichungstag der Anmeldung: **29.10.86 Patentblatt 86/44**

(84) Benannte Vertragsstaaten: **AT BE CH DE FR GB IT LI LU NL SE**

(71) Anmelder: **CIBA-GEIGY AG**
**Klybeckstrasse 141**
**CH-4002 Basel(CH)**

(72) Erfinder: **Schneider, Peter, Dr.**
**Bäumliackerstrasse 8**
**CH-4103 Bottmingen(CH)**
Erfinder: **Csendes, Ivan, Dr.**
**Finkenstrasse 9**
**CH-4104 Oberwil(CH)**
Erfinder: **Capraro, Hans Georg, Dr.**
**Habsburgerstrasse 60**
**CH-4310 Rheinfelden(CH)**

## (54) Heterocyclyl-penem-Verbindungen.

(57) 2-Heterocyclyl-6-hydroxyniederalkyl-2-penem-verbindungen der Formel

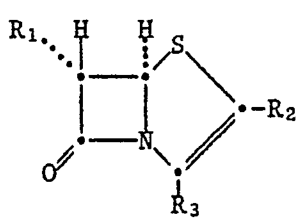

(I),

worin $R_1$ durch Hydroxy oder geschütztes Hydroxy substituiertes Niederalkyl ist, $R_2$ einen über ein Ringkohlenstoffatom an den Penem-Rest gebundenen monocyclischen 5-gliedrigen Heteroarylrest mit einem oder zwei Ringstickstoffatomen oder mit einem Ringstickstoffatom und einem zusätzlichen Ringsauerstoff-oder Ringschwefelatom darstellt, und $R_3$ Carboxyl oder funktionell abgewandeltes Carboxyl ist, und Salze von solchen Verbindungen der Formel I, die eine salzbildende Gruppe aufweisen, haben antibiotische Wirksamkeit. Die Verbindungen werden nach an sich bekannten Verfahren hergestellt.



Rank Xerox

# Heterocyclyl-penem-Verbindungen

Die Erfindung betrifft neue Heterocyclylpenem-Verbindungen, Verfahren zu ihrer Herstellung, pharmazeutische Präparate, die solche Verbindungen enthalten, und ihre Verwendung zur Herstellung von pharmazeutischen Präparaten oder als pharmakologisch wirksame Verbindungen.

Die Erfindung betrifft insbesondere 2-Heterocyclyl-6-hydroxyniederalkyl-2-penem-verbindungen der Formel

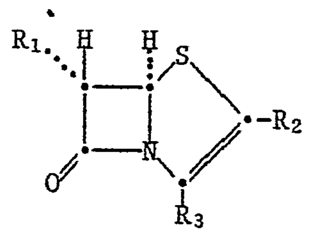

(I),

worin $R_1$ durch Hydroxy oder geschütztes Hydroxy substituiertes Niederalkyl ist, $R_2$ einen über ein Ringkohlenstoffatom an den Penem-Rest gebundenen monocyclischen 5-gliedrigen Heteroarylrest mit einem oder zwei Ringstickstoffatomen oder mit einem Ringstickstoffatom und einem zusätzlichen Ringsauerstoff-oder Ringschwefelatom darstellt, und $R_3$ Carboxyl oder funktionell abgewandeltes Carboxyl ist, und Salze von solchen Verbindungen der Formel I, die eine salzbildende Gruppe aufweisen.

Die vor-und nachstehend verwendeten Definitionen haben im Rahmen der vorliegenden Beschreibung vorzugsweise die folgenden Bedeutungen:

Ein über ein Ringkohlenstoffatom an den Penem-Rest gebundener monocyclischer 5-gliedriger Heteroarylrest $R_2$ mit einem oder zwei Ringstickstoffatomen oder mit einem Ringstickstoffatom und einem zusätzlichen Ringsauerstoff-oder Ringschwefelatom ist z.B. ein aza-, diaza-, oxaza-oder thiaza-cyclischer Rest aromatischen Charakters. Heteroarylreste $R_2$ sind beispielsweise über ein Ringkohlenstoffatom gebundenes Pyrrolyl, Diazolyl, wie Imidazolyl oder Pyrazolyl, Oxazolyl, Isoxazolyl, Thiazolyl oder Isothiazolyl.

Solche Reste $R_2$ sind unsubstituiert oder, beispielsweise zumindest an einem Ringkohlenstoffatom und gegebenenfalls zusätzlich an einen Ringstickstoffatom, substituiert. Geeignete Substituenten sind beispielsweise gegebenenfalls veräthertes oder verestertes Hydroxy, z.B. Hydroxy, Niederalkoxy, Niederalkanoyloxy oder Halogen, gegebenenfalls veräthertes Mercapto, z.B. Mercapto, Niederalkylthio oder Phenylthio, Niederalkyl, Hydroxyniederalkyl, Niederalkanoyloxyniederalkyl, Carboxyniederalkyl, Niederalkoxycarbonylniederalkyl, Carbamoylniederalkyl, Carbamoyloxyniederalkyl, Halogenniederalkyl, gegebenenfalls N-niederalkyliertes Aminoniederalkyl, z.B. Aminoniederalkyl, Niederalkylaminoniederalkyl oder Diniederalkylaminoniederalkyl, Niederalkanoylaminoniederalkyl, gegebenenfalls substituiertes Amino, z.B. Amino, Niederalkylamino, Diniederalkylamino oder Acylamino, wie Niederalkanoylamino, Acyl, wie gegebenenfalls funktionell abgewandeltes Carboxyl, z.B. Carboxyl, verestertes Carboxyl, wie Niederalkoxycarbonyl, gegebenenfalls substituiertes Carbamoyl, wie Carbamoyl oder N-mono-oder N,N-diniederalkyliertes Carbamoyl, oder Cyano, ferner Niederalkanoyl, gegebenenfalls durch Niederalkyl, Nitro, Niederalkoxy und/oder Halogen substituiertes Phenyl, und/oder Nitro. Solche Reste $R_2$ sind mono-oder auch poly-, in erster Linie mono-oder disubstituiert.

Funktionell abgewandeltes Carboxyl $R_3$ ist insbesondere unter physiologischen Bedingungen spaltbares, verestertes Carboxyl oder geschütztes Carboxyl $R_3'$.

Unter physiologischen Bedingungen spaltbare (metabolisierbare) veresterte Carboxylgruppen $R_3$ sind aus der Cephalosporin-, Penicillin-und Penemchemie bekannt. Geeignete Gruppen sind in erster Linie Acyloxymethoxycarbonylgruppen, worin Acyl z.B. den Rest einer organischen Carbonsäure, in erster Linie einer gegebenenfalls substituierten Niederalkancarbonsäure, bedeutet oder worin Acyloxymethyl den Rest eines Lactons bildet. Solche Gruppen sind z.B. Niederalkanoyloxymethoxycarbonyl, Aminoniederalkanoyloxymethoxycarbonyl, insbesondere α-Aminoniederalkanoyloxymethoxycarbonyl, und 4-Crotonolactonyl. Weitere unter physiologischen Bedingungen spaltbare veresterte Carboxylgruppen $R_3$ sind z.B. 5-Indanyloxycarbonyl, Phthalidyloxycarbonyl, 1-Niederalkoxycarbonyloxyniederalkoxycarbonyl, 1-Niederalkoyniederalkoxycarbonyl oder 2-Oxo-1, 3-dioxolen-4-ylmethoxycarbonyl, welches in 5-Stellung des Dioxolenringes gegebenenfalls durch Niederalkyl oder Phenyl substituiert ist.

In der vorliegenden Beschreibung bedeutet der im Zusammenhang mit Definitionen von Gruppen bzw. Verbindungen verwendete Ausdruck "Nieder", dass die entsprechenden Gruppe bzw. Verbindungen, sofern nicht ausdrücklich anders definiert, 1 bis 7, bevorzugt 1 bis 4, Kohlenstoffatome enthalten.

Durch Hydroxy substituiertes Niederalkyl $R_1$ ist insbesondere in α-Stellung zum Penem-Ringgerüst durch Hydroxy substituiertes Niederalkyl und bedeutet z.B. 1-Hydroxyprop-1-yl, 2-Hydroxyprop-2-yl, 1-Hydroxybut-1-yl oder 2-Hydroxybut-2-yl und insbesondere Hydroxymethyl oder 1-Hydroxyäthyl.

Niederalkoxy ist z.B. Methoxy oder Aethoxy, ferner auch n-Propoxy, Isopropoxy, n-Butoxy, Isobutoxy oder tert.-Butoxy, sowie n-Pentyloxy, n-Hexyloxy oder n-Heptyloxy.

Niederalkanoyloxy ist z.B. Acetoxy oder Propionyloxy.

Halogen ist z.B. Fluor, Chlor, Brom oder Jod.

Niederalkylthio ist z.B. Methylthio, Aethylthio, n-Propylthio, Isopropylthio oder n-Butylthio.

Niederalkyl ist z.B. n-Propyl, Isopropyl, n-Butyl, Isobutyl, sek.-Butyl oder tert.-Butyl, ferner n-Pentyl, n-Hexyl oder n-Heptyl und insbesondere Methyl oder Aethyl.

Hydroxyniederalkyl als Substituent eines Heteroaryl-Restes $R_2$ ist z.B. Hydroxymethyl, 2-Hydroxyäthyl oder 2,3-Dihydroxypropyl.

Niederalkanoyloxyniederalkyl ist z.B. Acetoxymethyl oder 2-Acetoxyäthyl.

Carboxyniederalkyl ist z.B. Carboxymethyl, 1-Carboxy-, 2-Carboxy-oder 1,2-Dicarboxyäthyl.

Niederalkoxycarbonylniederalkyl ist z.B. Methoxycarbonylmethyl, Aethoxycarbonylmethyl oder 2-Methoxycarbonyläthyl.

Carbamoylniederalkyl ist z.B. Carbamoylmethyl oder 2-Carbamoyläthyl, während Carbamoyloxyniederalkyl z.B. Carbamoyloxymethyl oder 2-Carbamoyloxyäthyl ist.

Halogenniederalkyl ist z.B. Chlormethyl, Brommethyl, 2-Chloräthyl oder 2,2-Dichloräthyl.

Aminoniederalkyl ist z.B. Aminomethyl oder 2-Aminoäthyl, während Niederalkylaminoniederalkyl z.B. Methylaminomethyl, Aethylaminomethyl, 2-Methylaminoäthyl oder 2-Aethylaminoäthyl und Diniederalkylaminoniederalkyl z.B. Dimethylaminomethyl, 2-Dimethylaminoäthyl oder 2-Diäthylaminoäthyl ist.

Niederalkanoylaminoniederalkyl ist z.B. Acetaminomethyl, 2-Acetaminoäthyl oder Formylaminomethyl.

Niederalkylamino ist z.B. Methylamino, Aethylamino, n-Propylamino, Isopropylamino oder n-Butylamino, während Diniederalkylamino z.B. Dimethylamino, Diäthylamino, Di-n-propylamino oder Diisopropylamino bedeutet.

Niederalkanoylamino ist z.B. Acetylamino oder Propionylamino.

Niederalkoxycarbonyl ist z.B. Methoxycarbonyl oder Aethoxycarbonyl.

N-Mono-niederalkyliertes Carbamoyl ist z.B. N-Methyl-, N-Aethyl-oder N-Propylcarbamoyl, während N,N-di-niederalkyliertes Carbamoyl z.B. N,N-Dimethyl-oder N,N-Diäthylcarbamoyl bedeutet.

Niederalkanoyl ist z.B. Formyl oder Acetyl.

Niederalkanoyloxymethoxycarbonyl ist z.B. Acetoxymethoxycarbonyl oder Pivaloyloxymethoxycarbonyl.

α-Aminoniederalkanoyloxymethoxycarbonyl ist z.B. Glycyloxymethoxycarbonyl, Valyloxymethoxycarbonyl oder Leucyloxymethoxycarbonyl.

1-Niederalkoxycarbonyloxyniederalkoxycarbonyl ist z.B. Aethoxycarbonyloxymethoxycarbonyl oder 1-Aethoxycarbonyloxyäthoxycarbonyl.

1-Niederalkoxyniederalkoxycarbonyl ist z.B. Methoxymethoxycarbonyl oder 1-Methoxyäthoxycarbonyl.

Bei einer 2-Oxo-1,3-dioxolen-4-ylmethoxy-Gruppe, welche in 5-Stellung des Dioxolenringes gegebenenfalls durch Niederalkyl oder Phenyl substituiert ist, handelt es sich vor allem um eine 5-Phenyl-und in erster Linie um eine 5-Methyl-2-oxo-1,3-dioxolen-4-ylmethoxy-Gruppe.

Heteroarylreste $R_2$ sind z.B. über ein Ringkohlenstoffatom an den Penem-Rest gebundenes unsubstituiertes oder durch Halogen, Niederalkyl und/oder Niederalkoxy substituiertes Pyrrolyl, wie 2-oder 3-Pyrrolyl, unsubstituiertes oder durch Amino, Niederalkylamino, Diniederalkylamino, Niederalkyl, Halogenniederalkyl, Hydroxyniederalkyl, Aminoniederalkyl, Diniederalkylaminoniederalkyl, Carboxyniederalkyl, Niederalkoxycarbonylniederalkyl, Carbamoylniederalkyl, Carbamoyloxyniederalkyl und/oder Niederalkoxy substituiertes Imidazolyl, wie 2-oder 4-Imidazolyl, oder Pyrazolyl, wie 3-oder 4-Pyrazolyl, unsubstituiertes oder durch Niederalkyl, Halogen, Niederalkoxy und/oder Phenyl substituiertes Oxazolyl, wie 2-, 4-oder 5-Oxazolyl, unsubstituiertes oder durch Amino, Niederalkylamino, Diniederalkylamino, Niederalkanoylamino, Niederalkyl, Hydroxyniederalkyl, Halogenniederalkyl, Aminoniederalkyl, Carboxyniederalkyl, Carbamoyloxyniederalkyl, Niederalkanoyl, Halogen, Carboxyl und/oder Nitro substituiertes Thiazolyl, wie 2-, 4- und 5-Thiazolyl, unsubstituiertes oder durch Niederalkyl und/oder Halogen substituiertes Isoxazolyl,

wie 3-, 4-oder 5-Isoxazolyl, oder unsubstituiertes oder durch Niederalkyl, Niederalkoxy, Halogen, Carboxyl, Amino, Cyano, Niederalkanoyl und/oder Nitro substituiertes Isothiazolyl, wie 4-oder 5-Isothiazolyl.

Bevorzugte Reste $R_2$ sind unsubstituiertes oder wie oben angegeben substituiertes Imidazolyl, 1,3,4-Triazolyl, Thiazolyl, Isothiazolyl und Oxazolyl.

Bevorzugte, unter physiologischen Bedingungen spaltbare veresterte Carboxylgruppen $R_3$ sind z.B. Niederalkanoyloxymethoxycarbonyl, z.B. Acetoxymethoxycarbonyl oder Pivaloyloxymethoxycarbonyl, und 1-Niederalkoxycarbonyloxy-niederalkoxycarbonyl, z.B. 1-Aethoxycarbonyloxyäthoxycarbonyl.

Die in den Verbindungen der Formel I vorhandenen funktionellen Gruppen, wie Hydroxy-, Carboxy-oder Aminogruppen, insbesondere die Hydroxygruppe im Rest $R_1$ und die Carboxylgruppe $R_3$, sind gegebenenfalls durch Schutzgruppen geschützt, die in der Penem-, Penicillin-, Cephalosporin-und Peptidchemie verwendet werden. Solche Schutzgruppen schützen die betreffenden funktionellen Gruppen vor unerwünschten Kondensationsreaktionen, Substitutionsreaktionen und dergleichen während der Synthese der Verbindung der Formel I aus ihren Vorstufen.

Solche Schutzgruppen sind leicht, d.h. ohne dass unerwünschte Nebenreaktionen stattfinden, beispielsweise solvolytisch, reduktiv oder auch unter physiologischen Bedingungen, abspaltbar.

Schutzgruppen dieser Art sowie ihre Einführung und Abspaltung sind beispielsweise beschrieben in

J.F.W. McOmie, "Protective Groups in Organic Chemistry", Plenum Press, London, New York, 1973,

T.W. Greene, "Protective Groups in Organic Synthesis", Wiley, New York, 1981,

"The Peptides", Vol. I, Schroeder und Luebke, Academic Press, London, New York, 1965 und

Houben-Weyl, "Methoden der Organischen Chemie", Band 15/1, Georg Thieme Verlag, Stuttgart, 1974.

In Verbindungen der Formel (I) kann eine Hydroxygruppe im Rest $R_1$, ferner eine im Rest $R_2$ vorhandene Hydroxygruppe, beispielsweise durch Acylreste geschützt sein. Geeignete Acylreste sind z.B. gegebenenfalls durch Halogen substituiertes Niederalkanoyl, z.B. Acetyl, Dichloracetyl oder Trifluoracetyl, gegebenenfalls durch Nitro substituiertes Benzoyl, z.B. Benzoyl, 4-Nitrobenzoyl oder 2,4-Dinitrobenzoyl, gegebenenfalls durch Halogen substituiertes Niederalkoxycarbonyl, z.B. 2-Bromäthoxycarbonyl oder 2,2,2-Trichloräthoxycarbonyl, Niederalkenyloxycarbonyl, z.B. Allyloxycarbonyl, Niederalkenyloxyoxalyl, z.B. Allyloxyoxalyl, oder gegebenenfalls durch Nitro substituiertes Phenylniederalkoxycarbonyl, z.B. Benzyloxycarbonyl oder 4-Nitrobenzyloxycarbonyl. Weitere geeignete Hydroxyschutzgruppen sind z.B. trisubstituiertes Silyl, wie Triniederalkylsilyl, z.B. Trimethylsilyl, Dimethyl-(2,3-dimethylbutyl)-silyl oder tert.-Butyl-dimethylsilyl, und 2-Oxa-oder 2-Thiacyclo alkyl mit 5 bis 7 Kohlenstoffatomen, z.B. 2-Tetrahydropyranyl oder 2-Tetrahydrofuranyl. Bevorzugt als Hydroxyschutzgruppe ist Triniederalkylsilyl und Niederalkenyloxycarbonyl.

Eine Carboxylgruppe $R_3$, ferner auch eine im Rest $R_2$ vorhandene Carboxylgruppe, ist üblicherweise in veresterter Form geschützt, wobei die Estergruppe unter schonenden Bedingungen, z.B. unter schonend reduktiven, wie Hydrogenolytischen, oder schonend solvolytischen, wie acidolytischen oder insbesondere basisch oder neutral hydrolytischen Bedingungen, leicht spaltbar ist.

Solche veresterten Carboxylgruppen enthalten als veresternde Gruppen in erster Linie in 1-Stellung verzweigte oder in 1-oder 2-Stellung geeignet substituierte Niederalkylgruppen. Geeignete in veresterter Form vorliegende Carboxylgruppen sind unter anderen Niederalkoxycarbonyl, z.B. Methoxycarbonyl, Aethoxycarbonyl oder tert.-Butoxycarbonyl, gegebenenfalls durch Nitro oder Niederalkoxy, wie Methoxy, substituiertes Benzyloxycarbonyl, z.B. 4-Nitrobenzyloxycarbonyl oder 4-Methoxybenzyloxycarbonyl, Niederalkanoylmethoxycarbonyl, wie Acetonyloxycarbonyl, Benzoylmethoxycarbonyl, worin die Benzoylgruppe vorzugsweise gegebenenfalls, z.B. durch Halogen, wie Brom, substituiert ist, z.B. Phenacyloxycarbonyl, Halogenniederalkoxycarbonyl, wie 2-Halogenniederalkoxycarbonyl, z.B. 2,2,2-Trichloräthoxycarbonyl oder 2-Bromäthoxycarbonyl, Niederalkenyloxycarbonyl, z.B. Allyloxycarbonyl, oder in 2-Stellung durch Niederalkylsulfonyl, Cyano oder trisubstituiertes Silyl, wie Triniederalkylsilyl oder Triphenylsilyl, substituiertes Aethoxycarbonyl, z.B. 2-Methylsulfonyläthoxycarbonyl, 2-Cyanoäthoxycarbonyl, 2-Trimethylsilyläthoxycarbonyl oder 2-(Di-n-butylmethyl-silyl)-äthoxycarbonyl. Weitere in veresterter Form vorliegende geschützte Carboxylgruppen sind entsprechende organische Silyloxycarbonylgruppen. In diesen enthält das Siliciumatom vorzugsweise Niederalkyl, insbesondere Methyl oder

Aethyl, ferner Niederalkoxy, z.B. Methoxy, als Substituenten. Geeignete Silylgruppen sind in erster Linie Triniederalkylsilylgruppen, insbesondere Trimethylsilyl oder Dimethyl-tert.-butylsilyl.

Bevorzugte geschützte Carboxylgruppen $R_3'$ sind die 4-Nitrobenzyloxycarbonyl-, Niederalkenyloxycarbonyl-, insbesondere Allyloxycarbonyl-, und die in 2-Stellung durch Triniederalkylsilyl, z.B. Trimethylsilyl oder Di-n-butylmethylsilyl, substituierte Aethoxycarbonylgruppe.

Eine geschützte Aminogruppe im Rest $R_2$ kann beispielsweise in Form einer leicht spaltbaren Acylamino-oder Silylaminogruppe oder als Nitro- oder Azidogruppe vorliegen.

In einer entsprechenden Acylaminogruppe ist Acyl beispielsweise der Acylrest einer organischen Säure mit z.B. bis zu 18 Kohlenstoffatomen, insbesondere einer gegebenenfalls z.B. durch Halogen oder Phenyl, substituierten Alkancarbonsäure oder insbesondere eines Kohlensäurehalbesters. Solche Acylgruppen sind beispielsweise Halogenniederalkanoyl, wie 2-Halogenacetyl, insbesondere 2,2,2-Trifluor-oder 2,2,2-Trichloracetyl, Niederalkenyloxycarbonyl, z.B. Allyloxycarbonyl, gegebenenfalls in 1-oder 2-Stellung substituiertes Niederalkoxycarbonyl, wie Niederalkoxycarbonyl, z.B. tert.-Butoxycarbonyl, gegebenenfalls substituiertes Benzyloxycarbonyl, z.B. 4-Nitrobenzyloxycarbonyl, 2-Halogenniederalkoxycarbonyl, z.B. 2,2,2-Trichloräthoxycarbonyl oder 2-Bromäthoxycarbonyl oder 2-(trisubstituiertes Silyl)-äthoxycarbonyl, wie 2-Triniederalkylsilyläthoxycarbonyl, z.B. 2-Trimethylsilyläthoxycarbonyl oder 2-(Di-n-butylmethyl-silyl)-äthoxycarbonyl.

Eine Silylaminogruppe ist in erster Linie eine organische Silylaminogruppe, worin das Siliciumatom vorzugsweise Niederalkyl, z.B. Methyl, Aethyl, n-Butyl oder tert.-Butyl, ferner Niederalkoxy, z.B. Methoxy, als Substituenten enthält. Entsprechende Silylgruppen sind in erster Linie Triniederalkylsilyl, insbesondere Trimethylsilyl, ferner Dimethyl-tert.-butylsilyl.

Bevorzugte geschützte Aminogruppen sind z.B. Azido, Nitro, Niederalkenyloxycarbonylamino, z.B. Allyloxycarbonylamino, und gegebenenfalls durch Nitro substituiertes Benzyloxycarbonylamino.

Salze von erfindungsgemässen Verbindungen sind in erster Linie pharmazeutisch annehmbare, nichttoxische Salze von Verbindungen der Formel I. Solche Salze werden beispielsweise von den in Verbindungen der Formel I vorhandenen sauren Gruppen, z.B. Carboxyl-und Sulfogruppen, gebildet und sind in erster Linie Metall-oder Ammoniumsalze, wie Alkalimetall-und Erdalkalimetall-, z.B. Natrium-, Kalium-, Magnesium-oder Kalziumsalze, sowie Ammoniumsalze mit Ammoniak oder geeigneten organischen Aminen, wie Niederalkylaminen, z.B. Triäthylamin, Hydroxyniederalkylaminen, z.B. 2-Hydroxyäthylamin, Bis-(2-hydroxyäthyl)-amin oder Tris-(2-hydroxyäthyl)-amin, basischen aliphatischen Estern von Carbonsäuren, z.B. 4-Aminobenzoesäure-2-diäthylaminoäthylester, Niederalkylenaminen, z.B. 1-Aethylpiperidin, Cycloalkylaminen, z.B. Dicyclohexylamin, oder Benzylaminen, z.B. N,N'-Dibenzyläthylendiamin, Dibenzylamin oder N-Benzyl-$\beta$-phenäthylamin. Verbindungen der Formel I mit einer basischen Gruppe, z.B. mit einer Aminogruppe, können Säureadditionssalze, z.B. mit anorganischen Säuren, wie Salzsäure, Schwefelsäure oder Phosphorsäure, oder mit geeigneten organischen Carbon-oder Sulfonsäure, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure bilden. Verbindungen der Formel I mit einer sauren und mit einer basischen Gruppe können auch in Form von inneren Salzen, d.h. in zwitterionischer Form, vorliegen.

Zur Isolierung oder Reinigung können auch pharmazeutisch ungeeignete Salze Verwendung finden. Zur therapeutischen Anwendung gelangen nur die pharmazeutisch annehmbaren, nicht-toxischen Salze, die deshalb bevorzugt sind.

Die Penem-Verbindungen der Formel I können im Substituenten $R_1$ ein zusätzliches Chiralitätszentrum besitzen. Beispielsweise kann 1-Hydroxyäthyl als Substituent $R_1$ in der R-, in der S- oder in der racemischen R,S-Konfiguration vorliegen. In bevorzugten Penem-Ver bindungen der Formel I weist der Rest $R_1$, welcher ein asymmetrisches Kohlenstoffatom besitzt, insbesondere 1-Hydroxyäthyl, die R-Konfiguration auf.

Die Erfindung betrifft hauptsächlich Verbindungen der Formel I, worin $R_1$ durch Hydroxy oder geschütztes Hydroxy substituiertes Niederalkyl ist, $R_2$ einen über ein Ringkohlenstoffatom an den Penem-Rest gebundenen aza-, diaza-, oxaza-oder thiazacyclischen Rest aromatischen Charakters darstellt, der unsubstituiert oder zumindest an einem Ringkohlenstoffatom und gegebenenfalls zusätzlich an einem Ringstickstoffatom durch Hydroxy, Niederalkoxy, Niederalkanoyloxy, Halogen, Mercapto, Niederalkylthio, Phenylthio, Niederalkyl, Hydroxyniederalkyl, Niederalkanoyloxyniederalkyl, Carboxyniederalkyl, Niederalkoxycarbonylniederalkyl, Carbamoylniederalkyl, Carbamoyloxyniederalkyl, Halogenniederalkyl, gegebenenfalls N-niederalkyliertes Aminoniederalkyl, Niederalkanoylaminoniederalkyl, Amino, Niederalkylamino, Diniederalkylamino, Niederalkanoylamino, Carboxyl, Niederalkoxycarbonyl, Carbamoyl, N-mono-oder N,N-diniederalkyliertes

Carbamoyl, Cyano, Niederalkanoyl, gegebenenfalls durch Niederalkyl, Nitro, Niederalkoxy und/oder Halogen substituiertes Phenyl und/oder Nitro substituiert ist, und $R_3$ Carboxyl, unter physiologischen Bedingungen spaltbares verestertes Carboxyl oder geschütztes Carboxyl $R_3'$ bedeutet, und Salze von Verbindungen der Formel I, die eine salzbildende Gruppe aufweisen.

Die Erfindung betrifft vor allem Verbindungen der Formel I, worin $R_1$ durch Hydroxy substituiertes Niederalkyl ist, $R_2$ über ein Ringkohlenstoffatom an den Penem-Rest gebundenes, unsubstituiertes oder durch Amino, Niederalkyl und/oder Aminoniederalkyl substituiertes Imidazolyl oder Pyrazolyl, unsubstituiertes oder durch Niederalkyl und/oder Niederalkoxy substituiertes Oxazolyl, unsubstituiertes oder durch Amino, Niederalkylamino, Diniederalkylamino, Niederalkanoylamino, Niederalkyl, Aminoniederalkyl, Hydroxyniederalkyl, Halogenniederalkyl, Carbamoyloxyniederalkyl und/oder Halogen substituiertes Thiazolyl, oder unsubstituiertes oder durch Niederalkyl, substituiertes Isothiazolyl, darstellt, und $R_3$ Carboxyl oder unter physiologischen Bedingungen spaltbares verestertes Carboxyl bedeutet, und Salze von Verbindungen der Formel I, die eine salzbildende Gruppe aufweisen.

Die Erfindung betrifft in erster Linie Verbindungen der Formel I, worin $R_1$ in α-Stellung durch Hydroxy substituiertes Niederalkyl bedeutet, $R_2$ durch Amino und/oder Niederalkyl substituiertes Imidazol-4-yl, durch Amino, Niederalkyl, Hydroxyniederalkyl, Halogenniederalkyl und/oder Aminoniederalkyl substituiertes Thiazol-4-yl oder -5-yl, oder durch Amino substituiertes Isothiazol-4-yl darstellt, und $R_3$ Carboxyl oder unter physiologischen Bedingungen spaltbares verestertes Carboxyl ist, und Salze von Verbindungen der Formel I, die eine salzbildende Gruppe aufweisen.

Die Erfindung betrifft bevorzugt Verbindungen der Formel I, worin $R_1$ Hydroxymethyl oder 1-Hydroxyäthyl ist, $R_2$ an einem Ringkohlenstoffatom durch Niederalkyl, Amino und/oder Aminoniederalkyl substituiertes Thiazol-4-yl oder Thiazol-5-yl bedeutet und $R_3$ Carboxyl oder unter physiologischen Bedingungen spaltbares verestertes Carboxyl bedeutet, und Salze von solchen Verbindungen, welche eine salzbildende Gruppe aufweisen.

Die Erfindung betrifft vor allem die in den Beispielen genannten Verbindungen der Formel I und deren Salze, insbesondere pharmazeutisch annehmbaren Salze.

Die Verbindungen der vorliegenden Erfindung können nach an sich bekannten Verfahren hergestellt werden.

Die neuen Verbindungen werden z.B. hergestellt, indem man

a. eine Ylid-Verbindung der Formel

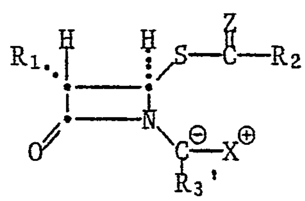

(II),

worin $R_1$ und $R_2$ die unter Formel I angegebenen Bedeutungen haben, $R_3'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b. eine Verbindung der Formel

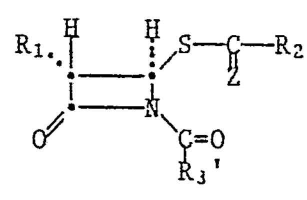

(III),

worin $R_1$ und $R_2$ die unter Formel I angegebenen Bedeutungen haben, Z die unter Formel II angegebene Bedeutung hat und $R_3'$ eine geschützte Carboxylgruppe darstellt, mit einer organischen Verbindung des dreiwertigen Phosphors behandelt, und, wenn erwünscht oder notwendig, in einer erhältlichen Verbindung der Formel I eine geschützte funktionnelle Gruppe in die freie funktionelle Gruppe überführt, und/oder, wenn erwünscht, in einer erhältlichen Verbindung der Formel I eine freie Carboxylgruppe $R_3$ in eine unter physiologischen Bedingungen spaltbare veresterte Carboxylgruppe überführt, und/oder, wenn erwünscht in einer erhältlichen Verbindung der Formel I einen Rest $R_2$ in einen anderen Rest $R_2$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

In den Ausgangsverbindungen der Formeln II und III sind funktionelle Gruppen, wie eine freie Hydroxygruppe im Rest $R_1$ sowie weitere im Rest $R_2$ enthaltene funktionelle Gruppen, vorzugsweise durch konventionelle Schutzgruppen, z.B. durch die oben genannten, geschützt.

a) Cyclisierung der Verbindung der Formel II

Die Gruppe X⊕ im Ausgangsmaterial der Formel II ist eine der bei Wittig-Kondensationsreaktionen gebräuchlichen Phosphonio-oder Phosphonogruppen, insbesondere eine Triaryl-, z.B. Triphenyl-, oder Triniederalkyl-, z.B. Tri-n-butylphosphoniogruppe, oder eine durch Niederalkyl, z.B. Aethyl, zweifach veresterte Phosphonogruppe, wobei das Symbol X⊕ für den Fall der Phosphonogruppe zusätzlich das Kation einer starken Base, insbesondere ein geeignetes Metall-, wie Alkalimetallion, z.B. das Lithium-, Natrium-oder Kaliumion, umfasst. Bevorzugt als Gruppe X⊕ sind einerseits Triphenylphosphonio und andererseits Diäthylphosphono zusammen mit einem Alkalimetall-, z.B. dem Natriumion.

In Phosphonio-Verbindungen der Formel II wird die negative Ladung durch die positiv geladene Phosphoniogruppe neutralisiert. In Phosphono-Verbindungen der Formel II wird die negative Ladung durch das Kation einer starken Base neutralisiert, das je nach Herstellungsweise des Phosphono-Ausgangsmaterials z.B. ein Alkalimetall-, z.B. Natrium-, Lithium-oder Kaliumion, sein kann. Die Phosphono-Ausgangsstoffe werden daher als Salze in die Reaktion eingesetzt.

Der Ringschluss kann spontan, d.h. bei der Herstellung der Ausgangsstoffe, oder durch Erwärmen, z.B. in einem Temperaturbereich von etwa 30°C bis 160°C, vorzugsweise von etwa 50°C bis etwa 100°C, erfolgen. Die Reaktion wird vorzugsweise in einem geeigneten inerten Lösungsmittel, wie in einem aliphatischen, cycloaliphatischen oder aromatischen Kohlenwasserstoff, z.B. Cyclohexan, Benzol oder Toluol, einem halogenierten Kohlenwasserstoff, z.B. Methylenchlorid, einem Aether, z.B. Diäthyläther, einem cyclischen Aether, z.B. Dioxan oder Tetrahydrofuran, einem Carbonsäureamid, z.B. Dimethylformamid, einem Diniederalkylsulfoxid, z.B. Dimethylsulfoxid, oder einem Nieder alkanol, z.B. Aethanol, oder in einem Gemisch davon, und, falls notwendig, in einer Inertgas-, z.B. Stickstoffatmosphäre, durchgeführt.

b. Cyclisierung der Verbindung der Formel III

Eine organische Verbindung des dreiwertigen Phosphors leitet sich z.B. von phosphoriger Säure ab und ist insbesondere ein Ester derselben mit einem Niederalkanol, z.B. Methanol oder Aethanol, und/oder einer gegebenenfalls substituierten aromatischen Hydroxyverbindung, z.B. Phenol oder Brenzcatechin, oder ein Amidester derselben der Formel $P(OR_a)-N(R_b)_2$, worin $R_a$ und $R_b$ unabhängig voneinander Niederalkyl, z.B. Methyl, oder Aryl, z.B. Phenyl, bedeuten. Bevorzugte Verbindungen des dreiwertigen Phosphors sind Triniederalkylphosphite, z.B. Trimethylphosphit oder Triäthylphosphit.

Die Reaktion wird vorzugsweise in einem inerten Lösungsmittel, wie einem aromatischen Kohlenwasserstoff, z.B. Benzol oder Toluol, einem Aether, z.B. Dioxan oder Tetrahydrofuran, oder einem halogenierten Kohlenwasserstoff, z.B. Methylenchlorid oder Chloroform, bei einer Temperatur von etwa 20° bis 140°C, bevorzugt von etwa 20° bis etwa 110°C, durchgeführt, wobei man 1 Moläquivalent einer Verbindung der Formel III mit mindestens 2 Moläquivalent der Phosphorverbindung umsetzt. Vorzugsweise legt man die Verbindung der Formel III in einem inerten Lösungsmittel vor und tropft die Phosphorverbindung, vorzugsweise in dem gleichen inerten Lösungsmittel gelöst, hinzu.

In einer bevorzugten Ausführungsform des Verfahrens stellt man das Ausgangsmaterial der Formel III wie weiter unten angegeben her und setzt es ohne Isolierung aus dem Reaktionsgemisch mit der organischen. Verbindung des dreiwertigen Phosphors um, wobei die Endprodukte der Formel I entstehen.

Bevorzugt werden solche Ausgangsmaterialien der Formeln II und III verwendet, die zu den eingangs als besonders bevorzugt genannten Verbindungen der Formel I führen.

In einer erhältlichen Verbindung der Formel I, worin eine oder mehrere funktionelle Gruppen geschützt sind, können diese, z.B. geschützte Carboxyl-, Hydroxy-, Amino-, und/oder Sulfogruppen, in an sich bekannter Weise mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

In einer verfahrensgemäss erhältlichen Verbindung der Formel I, worin $R_3$ eine geschützte Carboxylgruppe bedeutet und/oder worin der Rest $R_2$ geschütztes Carboxyl als Substituenten enthält, kann die geschützte Carboxylgruppe in an sich bekannter Weise freigesetzt werden. So kann man tert.-Niederalkoxycarbonyl oder in 2-Stellung durch eine trisubstituierte Silylgruppe substituiertes Niederalkoxycarbonyl z.B. durch Behandeln mit einer Carbonsäure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, gespalten werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zinn, oder Metallsalz, wie einem Chrom-II-Salz, z.B. Chrom-II-chlorid, üblicherweise in Gegenwart eines Wasserstoffabgebenden Mittels, das zusammen mit dem Metall nascierenden Wasserstoff zu erzeugen vermag, wie einer geeigneten Carbonsäure, z.B. einer gegebenenfalls, z.B. durch Hydroxy, substituierten Niederalkancarbonsäure, z.B. Essigsäure, Ameisensäure oder Glykolsäure, oder eines Alkohols oder Thiols, wobei man vorzugsweise Wasser zugibt, in freies Carboxyl überführen. Die Abspaltung einer Allylschutzgruppe kann z.B. durch Umsetzung mit einer Verbindung des Palladiums, z.B. Tetrakis-(triphenylphosphin)palladium, gegebenenfalls in Gegenwart von Triphenylphosphin und unter Zusatz eines Allylgruppenakzeptors, wie einer Carbonsäure, z.B. 2-Aethylhexan säure, oder eines Salzes davon oder Tributylzinnhydrid oder Dimedon, erfolgen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Benzoylmethoxycarbonyl in freies Carboxyl umwandeln, wobei Benzoylmethoxycarbonyl durch Behandeln mit einem nucleophilen, vorzugsweise salzbildenden Reagenz, wie Natriumthiophenolat oder Natriumjodid, gespalten werden kann. Substituiertes 2-Silyläthoxycarbonyl kann auch durch Behandeln mit einem das Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetrabutylammoniumfluorid, in freies Carboxyl überführt werden. Mit einer organischen Silylgruppe, wie Triniederalkylsilyl, verestertes Carboxyl kann in üblicher Weise solvolytisch, z.B. durch Behandeln mit Wasser oder einem Alkohol, freigesetzt werden. Eine in 2-Stellung durch Niederalkylsulfonyl oder Cyano substituierte Niederalkoxycarbonylgruppe kann z.B. durch Behandeln mit einem basischen Mittel, wie einem Alkalimetall-oder Erdalkalimetallhydroxid oder -carbonat, z.B. Natrium-oder Kaliumhydroxid oder Natrium-oder Kaliumcarbonat, in freies Carboxyl übergeführt werden.

Andererseits können auch Verbindungen der Formel I, worin $R_3$ Carboxy, bedeutet, in Verbindungen der Formel I überführt werden, worin $R_3$ eine unter physiologischen Bedingungen spaltbare veresterte Carboxylgruppe darstellt. Solche Ester können z.B. durch Umsetzung eines gegebenenfalls in situ hergestellten Salzes der Säure mit einem reaktionsfähigen Ester eines entsprechenden Alkohols und einer starken anorganischen Säure, wie Schwefelsäure, oder einer starken organischen Sulfonsäure, wie 4-Toluolsulfonsäure, hergestellt werden. Ferner können Säurehalogenide, wie Chloride (hergestellt z.B. durch Behandeln mit Oxalylchlorid), aktivierte Ester, (gebildet z.B. mit N-Hydroxystickstoffverbindungen, wie N-Hydroxy succinimid) oder gemischte Anhydride (erhalten z.B. mit Halogenameisensäure-niederalkylester, wie Chlorameisensäureäthyl -oder Chlorameisensäureisobutylester, oder mit Halogenessigsäurehalogeniden, wie Trichloressigsäurechlorid) durch Umsetzen mit geeigneten

Alkoholen, gegebenenfalls in Gegenwart einer Base, wie Pyridin, in eine veresterte Carboxylgruppe übergeführt werden. Ferner kann man in Verbindungen der Formel I, die eine in veresterter Form geschützte Carboxylgruppe enthalten, die Carboxylschutzgruppe wie oben beschrieben abspalten und eine entstandene Verbindung der Formel I mit einer freien Carboxylgruppe oder ein Salz davon durch Umsetzen mit dem reaktionsfähige Ester eines entsprechenden Alkohols in eine Verbindung der Formel I überführen, worin $R_3$ eine unter physiologischen Bedingungen spaltbare veresterte Carboxylgruppe darstellt.

In verfahrensgemäss erhältlichen Verbindungen der Formel I, worin der Rest $R_1$ und/oder gegebenenfalls der Rest $R_2$ geschütztes Hydroxyl als Substituenten enthält, kann die geschützte Hydroxygruppe in an sich bekannter Weise in die freie Hydroxygruppe überführt werden. Beispielsweise wird eine durch eine geeignete Acylgruppe oder eine organische Silylgruppe geschützte Hydroxygruppe wie eine entsprechend geschützte Aminogruppe freigesetzt (s.u.); eine Triniederalkylsilylgruppe z.B. auch mit Tetrabutylammoniumfluorid und Essigsäure (unter diesen Bedingungen werden durch trisubstituiertes Silyläthoxy geschützte Carboxygruppen nicht gespalten).

In einer erfindungsgemäss erhältlichen Verbindung der Formel I mit einer geschützten Aminogruppe kann diese in an sich bekannter Weise, z.B. je nach Art der Schutzgruppe, vorzugsweise mittels Solvolyse oder Reduktion, in die freie Aminogruppe übergeführt werden. Beispielsweise kann 2-Halogenniederalkoxycarbonylamino (gegebenenfalls nach Umwandlung einer 2-Bromniederalkoxycarbonylaminogruppe in eine 2-Jodniederalkoxycarbonylaminogruppe), 4-Nitrobenzyloxycarbonylamino durch Behandeln mit einem geeigneten chemischen Reduktionsmittel, wie Zink in Gegenwart einer geeigneten Carbonsäure, wie wässriger Essigsäure, oder katalytisch mit Wasserstoff in Gegenwart eines Palladiumkatalysators oder durch Behandeln mit einem Alkali metall-, z.B. Natriumdithionit gespalten werden. Gegebenenfalls substituiertes Benzyloxycarbonylamino kann z.B. mittels Hydrogenolyse, d.h. durch Behandeln mit Wasserstoff in Gegenwart eines geeigneten Hydrierkatalysators, wie eines Palladiumkatalysators, und Allyloxycarbonylamino durch Umsetzen mit einer Verbindung des Palladiums, z.B. Tetrakis-(triphenylphosphin)palladium, gegebenenfalls in Gegenwart von Triphenylphosphin und in Gegenwart eines Allylgruppenakzeptors, wie einer Carbonsäure, z.B. 2-Aethylhexansäure, oder eines Salzes davon oder Tributylzinnhydrid oder Dimedon, gespalten werden. Eine mit einer organischen Silylgruppe geschützte Aminogruppe kann z.B. mittels Hydrolyse oder Alkoholyse, eine durch 2-Halogenniederalkanoyl, z.B. 2-Chloracetyl, geschützte Aminogruppe durch Behandeln mit Thioharnstoff in Gegenwart einer Base oder mit einem Thiolatsalz, wie einem Alkalimetall-thiolat, des Thioharnstoffs und anschliessender Solvolyse, wie Alkoholyse oder Hydrolyse des entstandenen Kondensationsproduktes freigesetzt werden. Eine durch 2-substituiertes Silyläthoxycarbonyl geschützte Aminogruppe kann durch Behandeln mit einem Fluoridanion liefernden Salz der Fluorwasserstoffsäure, wie einem Alkalimetallfluorid, z.B. Natriumfluorid, in Anwesenheit eines makrocyclischen Polyäthers - ("Kronenäther") oder mit einem Fluorid einer organischen quaternären Base, wie Tetraniederalkylammoniumfluorid, z.B. Tetraäthylammoniumfluorid, in die freie Aminogruppe übergeführt werden. Eine in Form einer Azido-oder Nitrogruppe geschützte Aminogruppe wird z.B. durch Reduktion in freies Amino überführt, beispielsweise durch katalytische Hydrierung mit Wasserstoff in Gegenwart eines Hydrierkatalysators wie Platinoxid oder Palladium, oder durch Behandeln mit Zink in Gegenwart einer Säure, wie Essigsäure.

Eine geschützte, insbesondere veresterte, Sulfogruppe wird analog einer geschützten Carboxylgruppe freigesetzt.

In Verbindungen der Formel I kann man weiterhin einen Rest $R_2$ in einen anderen Rest $R_2$ überführen.

So kann beispielsweise in Verbindungen der Formel I, worin der Rest $R_2$ durch eine Carboxylgruppe substituiert ist, diese Carboxylgruppe nach an sich bekannten Verfahren in eine funktionell abgewandelte Carboxylgruppe, wie in eine veresterte Carboxylgruppe oder in gegebenenfalls substituiertes Carbamoyl, überführt werden. Beispielsweise erhält man durch Umsetzen einer Verbindung der Formel I, worin $R_2$ durch Carboxyl substituiertes Heteroaryl ist, mit einem Alkohol, insbesondere einem Niederalkanol, eine Verbindung der Formel I, worin $R_2$ durch verestertes Carboxyl, insbesondere Niederalkoxycarbonyl, substituiertes Heteroaryl ist, wobei man vorzugsweise in Gegenwart eines geeigneten Kondensationsmittels, z.B. eines Carbodiimids, arbeitet oder das entstehende Wasser durch azeotrope Destillation entfernt. Andererseits kann man Carboxylgruppen an Resten $R_2$ auch in reaktionsfähige funktionelle Derivate, wie gemischte Anhydride, z.B. Säurehalogenide, oder aktivierte Ester, überführen und diese durch Umsetzen mit einem Alkohol, z.B. Niederalkanol, Ammoniak oder einem primären oder sekundären Amin, z.B. einem Niederalkyl-oder Diniederalkylamin, in entsprechend veresterte oder amidierte Carboxylgruppen umwandeln, wobei man bei Verwendung von gemischten Anhydriden vorzugsweise in Gegenwart eines säurebindenden Mittels, wie eines aromatischen oder tertiären Amins oder eines Alkalimetall-oder Erdalkalimetallcarbonats, arbeitet.

Weist ein Heteroaryl-Rest $R_2$ eine Hydroxygruppe auf, so lässt sich diese auf üblichem Wege veräthern. Die Umsetzung zu den entsprechenden Niederalkyl-heteroaryl-äthern erfolgt beispielweise in Gegenwart von Basen, wie Alkalimetallhydroxiden oder -carbonaten, z.B. Natriumhydroxid oder Kaliumcarbonat, mit Hilfe von Diniederalkylsulfaten oder Niederalkylhalogeniden, oder mit Diazoniederalkanen, oder, in Gegenwat eines Dehydratisierungsmittels, beispielsweise Dicyclohexylcarbodiimid, mit Hilfe von Niederalkanolen. Weiterhin lässt sich Hydroxy in verestertes Hydroxy, z.B. Niederalkanoyloxy, umwandeln, beispielsweise durch Umsetzung mit dem reaktionsfähigen Derivat einer entsprechenden Niederalkancarbonsäure, z.B. Essigsäure, wie einem Anhydrid davon, z.B. dem symmetrischen Anhydrid davon oder einem gemischten Anhydrid mit einer Halogenwasserstoff säure, erforderlichenfalls in Gegenwart eines basischen Kondensationsmittels, wie eines Alkalimetallhydroxids oder -carbonats, oder einer Stickstoffbase, z.B. Pyridin. Die Umwandlung von Niederalkanoyloxy zu Hydroxy erfolgt beispielsweise durch Alkoholyse oder, vorzugsweise, Hydrolyse, z.B. durch basenkatalysierte Hydrolyse, z.B. in Gegenwart von Natriumhydroxid.

In Verbindungen der Formel I, worin $R_2$ durch Amino substituiertes Heteroaryl bedeutet, kann die Aminogruppe in eine substituierte Aminogruppe, z.B. eine Niederalkylamino-, Diniederalkylamino- oder Niederalkanoylaminogruppe, überführt werden. Die Ueberführung in eine Niederalkylamino- oder Diniederalkylaminogruppe erfolgt beispielsweise durch Reaktion mit einem reaktionsfähigen veresterten Niederalkanol, beispielsweise einem Niederalkylhalogenid oder -sulfonat, in Gegenwart eines basischen Kondensationsmittels, wie eines Hydroxids oder Carbonats eines Alkali-oder Erdalkalimetalls oder einer heteroaromatischen Stickstoffbase, z.B. Pyridin. In analoger Weise kann Amino durch Behandeln mit dem reaktionsfähigen funktionellen Derivat einer Niederalkancarbonsäure, z.B. dem entsprechenden Carbonsäurehalogenid, in Niederalkanoylamino umgewandelt werden. Verbindungen der Formel I mit einem substituierbaren Ringstickstoffatom im Rest $R_2$ können auf die gleiche Weise in Verbindungen der Formel I überführt werden, welche im Rest $R_2$ ein durch einen gegebenenfalls substituierten Niederalkylrest substituiertes Ringstickstoffatom enthalten. Die mit Hilfe der genannten Umsetzungen herstellbaren neuen Verbindungen der Formel I können daher im Rest $R_2$ ein entsprechend substituiertes sekundäres, tertiäres oder auch quaternäres, d.h. positiv geladenes, Stickstoffatom enthalten.

Salze von Verbindungen der Formel I mit salzbildenden Gruppen können in an sich bekannter Weise hergestellt werden. So kann man Salze von Verbindungen der Formel I mit einer freien Carboxyl-oder Sulfogruppe z.B. durch Behandeln mit Metallverbindungen, wie Alkalimetallsalzen von geeigneten organischen Carbonsäure, z.B. dem Natriumsalz der $\alpha$-Aethylcapronsäure, oder mit anorganischen Alkali-oder Erdalkalimetallsalzen, z.B. Natriumhydrogencarbonat, oder mit Ammoniak oder mit einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer geeigneten Säure oder einem geeigneten Anionenaustauscherreagenz. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall-und Ammoniumsalze z.B. durch Behandeln mit geeigneten Säuren und Säureadditionssalze z.B. durch Behandeln mit einem geeigneten basischen Mittel.

Erhaltene Gemische von isomeren Verbindungen können nach an sich bekannten Methoden in die einzelnen Isomere aufgetrennt werden. Beispielsweise kann man ein erhaltenes Racemat mit einem optisch aktiven Hilfsstoff reagieren lassen, das dabei entstandene Gemisch zweier diastereomerer Verbindungen mit Hilfe von geeigneten physikalisch-chemischen Methoden (z.B. fraktioniertes Kristallisieren, Adsorptionschromatographie) trennen und die einzelnen diastereomeren Verbindungen dann in die optisch aktiven Verbindungen spalten. Zur Trennung in Antipoden besonders geeignete Racemate sind solche, die eine saure Gruppe besitzen, wie z.B. Racemate von Verbindungen der Formel I, worin $R_3$ Carboxyl ist. Diese sauren Racemate können mit optisch aktiven Basen, z.B. Estern von optisch aktiven Aminosäuren, oder (-)-Brucin, (+)-Chinidin, (-)-Chinin, -(+)-Cinchonin, (+)-Dehydroabietylamin, (+)-und (-

)-Ephedrin, (+)-und (-)-1-Phenyl-äthylamin oder deren N-mono-oder N,N-dialkylierten Derivaten zu Gemischen, bestehend aus zwei diastereomeren Salzen, umgesetzt werden.

In Carboxylgruppen enthaltenden Racematen kann diese Carboxylgruppe auch durch einen optisch aktiven Alkohol, wie (-)-Menthol, (+)-Borneol, -(+)-oder (-()-2-Octanol verestert werden, worauf nach erfolgter Isolierung des gewünschten Diastereomeren die Carboxylgruppe freigesetzt wird.

Zur Racemattrennung kann auch eine vorhandene Hydroxygruppe mit optisch aktiven Säuren oder deren reaktionsfähigen, funktionellen Derivaten verestert werden, wobei sich diastereomere Ester bilden. Solche Säuren sind beispielsweise (-)-Abietinsäure, D(+)-und L(-)-Aepfelsäure, N-acylierte optisch aktive Aminosäuren, (+)-und (-)-Camphansäure, (+)-und (-)-Ketopinsäure, L(+)-Ascorbinsäure, (+)-Camphersäure, (+)-Campher-10-sulfonsäure(β), (+)-oder (-)-α-Bromcampher-β-sulfonsäure, D(-)-Chinasäure, D(-)-Isoascorbinsäure, D(-)-und L(+)-Mandelsäure, (+)-1-Menthoxyessigsäure, D(-)-und L(+)-Weinsäure und deren Di-O-Benzoyl-und Di-O-p-Tolylderivate.

Durch Umsetzung mit optisch aktiven Isocyanaten, wie mit (+)-oder (-)-1-Phenyläthylisocyanat, können Verbindungen der Formel (I), worin $R_3$ geschütztes Carboxy und $R_1$ durch Hydroxy substituiertes Niederalkyl bedeutet, in ein Gemisch diastereomerer Urethane umgewandelt werden.

Basische Racemate, z.B. Verbindungen der Formel I, worin der Rest $R_2$ durch Amino substituiert ist, können mit den genannten optischen aktiven Säuren diastereomere Salze bilden.

Die Spaltung der aufgetrennten Diastereomeren in die optisch aktiven Verbindungen der Formel I erfolgt ebenfalls nach üblichen Methoden. Aus den Salzen befreit man die Säuren oder die Basen z.B. durch Behandeln mit stärkeren Säuren bzw. Basen als die ursprünglich eingesetzten. Aus den Estern und Urethanen erhält man die gewünschten optisch aktiven Verbindungen beispielsweise nach alkalischer Hydrolyse oder nach Reduktion mit einem komplexen Hydrid, wie Lithiumaluminiumhydrid.

Die Auftrennung der Racemate in die optischen Antipoden kann auf einer beliebigen Verfahrensstufe, d.h. z.B. auch auf der Stufe der Ausgangsverbindungen der Formeln II oder III oder auf einer beliebigen Stufe der nachstehend beschriebenen Verfahren zur Herstellung der Ausgangsverbindungen der Formel II oder III, durchgeführt werden.

Bei allen nachträglichen Umwandlungen erhaltener Verbindungen der Formel I werden solche Reaktionen bevorzugt, die unter alkalischen oder insbesondere neutralen Bedingungen erfolgen.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird. Ferner können Ausgangsstoffe in Form von Derivaten verwendet oder in situ, gegebenenfalls unter den Reaktionsbedingungen, hergestellt werden.

Die Ausgangsverbindungen der Formeln II und III können, wie in dem folgenden Reaktionsschema I angegeben, hergestellt werden:

Reaktionsschema I

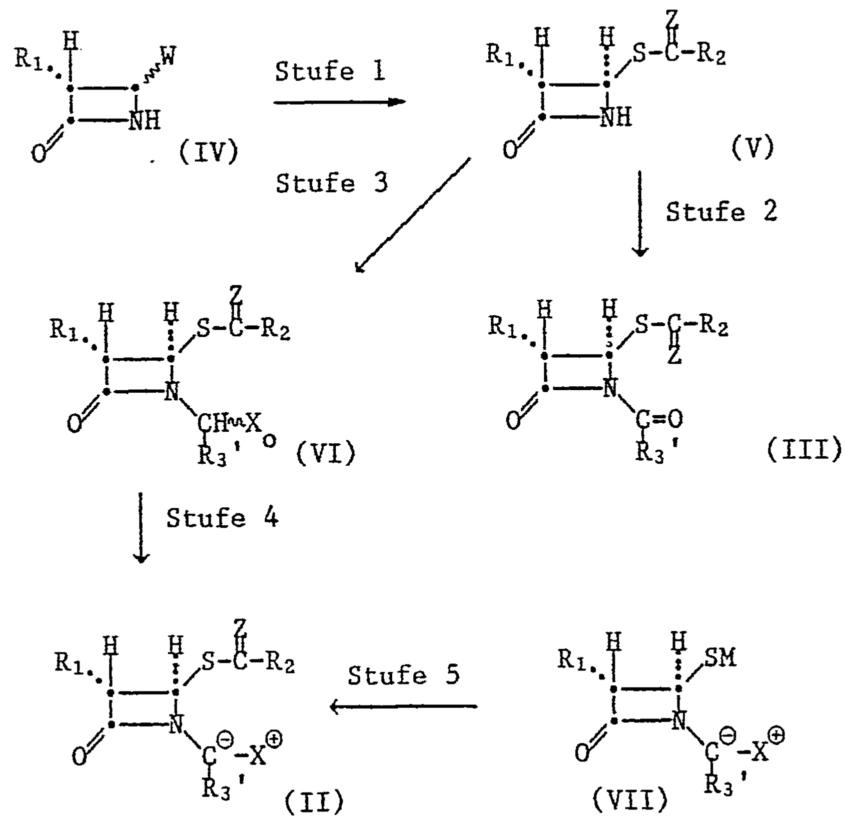


Stufe 1

Ein Thioazetidinon der Formel V wird erhalten, indem man eine Verbindung der Formel IV mit einer den Rest -S-C(=Z)-$R_2$ einführenden Verbindung umsetzt.

In einem Ausgangsmaterial der Formel IV ist W ein nucleofuger, durch die Gruppe -S-C(=Z)-$R_2$ ersetzbarer Rest. Solche Reste W sind beispielsweise Acyloxyreste, Sulfonylreste $R_o$-$SO_2$-, worin $R_o$ ein organischer Rest ist, Azido oder Halogen. In einem Acyloxyrest W ist Acyl z.B. der Rest einer organischen Carbonsäure und bedeutet beispielsweise Niederalkanoyl, z.B. Acetyl oder Propionyl, gegebenenfalls substituiertes Benzoyl, z.B. Benzoyl oder 2,4-Dinitrobenzoyl, oder Phenylniederalkanoyl, z.B. Phenylacetyl. In einem Sulfonylrest $R_o$-$SO_2$-ist $R_o$ beispielsweise gegebenenfalls durch Hydroxy substituiertes Niederalkyl, wie Methyl, Aethyl, 2-Hydroxyäthyl, 1-Hydroxyprop-2-yl oder 1-Hydroxy-2-methyl-prop-2-yl, Benzyl oder gegebenenfalls substituiertes Phenyl, wie Phenyl, 4-Bromphenyl oder 4-Methylphenyl. Ein Halogenrest W ist z.B. Brom, Jod oder insbesondere Chlor. W ist bevorzugt Methyl-oder 2-Hydroxyäthylsulfonyl, Acetoxy oder Chlor.

Eine den Rest -S-C(=Z)-$R_2$ einführende Verbindung ist beispielsweise eine Säure der Formel $R_2$-C(=Z)-SH oder insbesondere ein Salz, z.B. ein Alkalimetallsalz, wie das Natrium-oder Kaliumsalz, davon. Die Substitution kann in einem organischen Lösungsmittel, wie in einem Niederalkanol, z.B. Methanol oder Aethanol, einem Niederalkanon, z.B. Aceton, einem Niederalkancarbonsäureamid, z.B. Dimethylformamid, einem cyclischen Aether, z.B. Tetrahydrofuran oder Dioxan, oder in einem ähnlichen inerten Lösungsmittel durchgeführt werden. Die Reaktion wird üblicherweise bei Raumtemperatur durchgeführt, kann aber auch bei erhöhter oder erniedrigter Temperatur, z.B. bei etwa 0° bis etwa 40°C, durchgeführt werden. Durch Zugabe eines Salzes der Jodwasserstoffsäure oder der Thiocyansäure, z.B. eines Alkalimetall-, wie Natriumsalzes, kann die Reaktion beschleunigt werden.

Die eintretende Gruppe -S-C(=Z)-$R_2$ wird von dem Rest $R_1$ bevorzugt in die trans-Stellung dirigiert. Daher können sowohl (3S,4R)-als auch -(3S,4RS)-konfigurierte Ausgangsverbindungen der Formel IV eingesetzt werden. Obwohl die trans-Isomeren überwiegend gebildet werden, können doch gelegentlich auch geringe Mengen des cis-Isomeren entstehen. Die Abtrennung der cis-Isomeren erfolgt, wie oben beschrieben, nach konventionellen Methoden, insbesondere durch Chromatographie und/oder durch Kristallisation.

Geeignete Ausgangsverbindungen der Formel IV sind beispielsweise aus der Europäischen Patentanmeldung Nr. 82113, der Deutschen Offenlegungsschrift Nr. 3 224 055 oder der Deutschen Offenlegungsschrift 3 013 997 bekannt oder können in analoger Weise hergestellt werden. Sie können auch nach den in den Beispielen beschriebenen Verfahren hergestellt worden.

Stufe 2

Eine Ausgangsverbindung der Formel (III) wird erhalten, indem man ein Azetidinon der Formel (V) mit einer Säure der Formel $R_3$'-COOH oder insbesondere einem reaktionsfähigen Derivat, wie einem Ester oder Säurehalogenid, z.B. dem Säurechlorid, davon bei einer Temperatur von -50° bis 80°C, bevorzugt bei -20° bis 0°C, in einem inerten Lösungsmittel, wie einem der bei der Umsetzung von Verbindungen der Formel III zu Verbindungen der Formel I genannten, behandelt. Bei Verwendung eines Säurehalogenids arbeitet man vorzugsweise in Gegenwart eines säurebindenden Mittels, wie eines tertiären aliphatischen Amins, z.B. Triäthylamin, eines aromatischen Amins, z.B. Pyridin, oder insbesondere eines Alkalimetall-oder Erdalkalimetallcarbonats oder -hydrogencarbonats, z.B. Kaliumcarbonat oder Calciumcarbonat.

Stufe 3

Verbindungen der Formel VI, worin $X_0$ für eine reaktionsfähige veresterte Hydroxygruppe, insbesondere für Halogen, z.B. Chlor oder Brom, oder organisches Sulfonyloxy, z.B. Niederalkansulfonyloxy, wie Methansulfonyloxy, oder Arensulfonyloxy, z.B. Benzol-oder 4-Methylbenzolsulfonyloxy, steht, werden hergestellt, indem man eine Verbindung der Formel V mit einer Glyoxylsäure-Verbindung der Formel OHC-$R'_3$ oder einem geeigneten Derivat davon, wie einem Hydrat, Hemihydrat oder Halbacetal, z.B. einem Halbacetal mit einem Niederalkanol, z.B. Methanol oder Aethanol, umsetzt, und in einer erhaltenen Verbindung der Formel VI, worin $X_0$ für Hydroxy steht, die Hydroxygruppe in eine reaktionsfähige veresterte Hydroxygruppe umwandelt.

Die Verbindungen der Formel IV werden üblicherweise als Gemisch der beiden Isomeren -[bezüglich der Gruppierung -CH($R'_3$)$\sim X_0$] erhalten. Man kann aber auch die reinen Isomeren davon isolieren, z.B. durch Chromatographie.

Die Anlagerung der Glyoxylsäureester-Verbindung an das Stickstoffatom des Lactamrings in der Verbindung der Formel V findet bei Raumtemperatur oder, falls notwendig, unter Erwärmen, z.B. bei etwa 100°C, und zwar in Abwesenheit eines eigentlichen Kondensationsmittels statt. Bei Verwendung des Hydrats der Glyoxylsäure-Verbindung wird Wasser gebildet, das, wenn notwendig, durch Destillation, z.B. azeotrop, oder durch Verwendung eines geeigneten Dehydratationsmittels, wie eines Molekularsiebs, enfernt wird. Vorzugsweise arbeitet man in Gegenwart eines geeigneten Lösungsmittels, wie z.B. Dioxan, Toluol oder Dimethylformamid, oder Lösungsmittelgemisches, und, wenn erwünscht oder notwendig in der Atmosphäre eines Inertgases, wie Stickstoff.

Die Ueberführung einer Hydroxygruppe $X_0$ in eine reaktionsfähige veresterte Hydroxygruppe $X_0$ in einer Verbindung der Formel VI wird durch Behandeln mit einem geeigneten Veresterungsmittel durchgeführt, z.B. mit einem Thionylhalogenid, z.B. -chlorid, einem Phosphoroxyhalogenid, besonders -chlorid, einem Halogenphosphoniumhalogenid, wie Triphenylphosphoniumdibromid oder -dichlorid oder einem geeigneten organischen Sulfonsäurehalogenid, wie -chlorid, vorzugsweise in Gegenwart eines basischen, in erster Linie organischen basischen Mittels, wie einem aliphatischen tertiären Amins, z.B. Triäthylamin oder Diisopropylamin, oder einer heterocyclischen Base vom Pyridintyp, z.B. Pyridin oder Collidin. Vorzugsweise arbeitet man in Gegenwart eines geeigneten Lösungsmittels, z.B. Dioxan oder Tetrahydrofuran, oder eines Lösungsmittelgemisches, wenn notwendig unter Kühlen, z.B. bei etwa -30° bis etwa 30°C, und gegebenenfalls in der Atmosphäre eines Inertgases, wie Stickstoff.

Stufe 4

Das Ausgangsmaterial der Formel (II) wird erhalten, indem man eine Verbindung der Formel -(VI) mit einer geeigneten Phosphin-Verbindung, wie einem Triniederalkylphosphin, z.B. Tri-n-butylphosphin, oder einem Triaryl-phosphin, z.B. Triphenylphosphin, oder mit einer geeigneten Phosphit-Verbindung, wie einem Triniederalkylphosphit, z.B. Triäthylphosphit, oder einem Alkalimetalldiniederalkylphosphit, z.B. -diäthylphosphit, behandelt, und in einer erhältlichen Verbindung der Formel (II) den Rest $R_2$ gegebenenfalls gegen einen anderen Rest $R_2$ austauscht.

Die Umwandlung der Verbindung der Formel -(VI) in die Verbindung der Formel (II) wird vorzugsweise in Gegenwart eines geeigneten inerten Lösungsmittels, wie eines Kohlenwasserstoffs, z.B.

Cyclohexan oder Benzol, oder eines Aethers, z.B. Dioxan, oder eines Lösungsmittelgemisches vorgenommen. Je nach Reaktionsfähigkeit arbeitet man unter Kühlen oder bei erhöhter Temperatur, etwa zwischen -10° und +100°C, bevorzugt bei etwa 20° bis 80°C, und/oder in der Atmosphäre eines inerten Gases, wie Stickstoff. Zur Verhinderung oxidativer Prozesse können katalytische Mengen eines Antioxidans, z.B. Hydrochinon, zugesetzt werden.

Dabei arbeitet man üblicherweise in Gegenwart eines basischen Mittels, wie einer organischen Base, z.B. eines Amins, wie Triäthylamin, Diisopropyläthylamin, Pyridin, Lutidin, oder "Polystyrol-Hünigbase", oder einer anorganischen Base, z.B. eines Alkalimetallcarbonats, z.B. Natrium-oder Kaliumcarbonat, wobei das primär entstandene Phosphoniumsalz der Formel

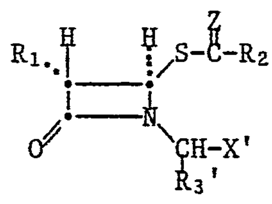

(IIa),

worin X' eine Phosphonogruppe oder eine Phosphoniogruppe zusammen mit einem Anion, je nach Bedeutung des Restes $X_o$ (vgl. Formel VI) z.B. Chlorid, bedeutet, in das Ylid-Ausgangsmaterial der Formel II umgewandelt wird. Man kann aber auch in Abwesenheit einer Base arbeiten und eine Verbindung der Formel (IIa), insbesondere eine entsprechende Phosphono-Verbindung, isolieren und diese bei der Herstellung der Endprodukte der Formel (I) in situ in das Ausgangsmaterial der Formel II überführen.

Stufe 5

Eine Verbindung der Formel (II) kann weiterhin erhalten werden, indem man ein Mercaptid der Formel (VII), worin M für ein Metallkation steht, mit einem den Rest $R_2$-C(=Z)-einführenden Acylierungsmittel behandelt.

In dem Ausgangsmaterial der Formel (VII) ist das Metallkation M beispielsweise ein Kation der Formel $M^+$ oder $M^{2+}/2$, wobei $M^+$ insbesondere für ein Silberkation und $M^{2+}$ insbesondere für das zweiwertige Kation eines geeigneten Uebergangmetalls, z.B. Kupfer, Blei oder Quecksilber, steht.

Ein den Rest $R_2$-C(=Z)-einführendes Acylierungsmittel ist z.B. die Säure $R_2$-C(=Z)-OH oder insbesondere ein reaktionsfähiges funktionelles Derivat davon, wie ein Säurehalogenid, z.B. Chlorid oder Bromid, Azid oder Anhydrid davon. Die Acylierung erfolgt, wenn ein reaktionsfähiges funktionelles Derivat der Säure der Formel $R_2$-C(=Z)-OH, z.B. das Säurechlorid, eingesetzt wird, in einem inerten Lösungsmittel, wie einem chlorierten Kohlenwasserstoff, z.B. Methylenchlorid, oder einem Aether, z.B. Diäthyläther oder Dioxan, bei Raumtem peratur oder unter Erhitzen oder Kühlen, z.B. in einem Temperaturbereich von ca. -50° bis ca. +60°C, insbesondere bei ca. -30° bis ca. +20°C.

Die Ausgangsverbindungen der Formel (VII) können beispielsweise hergestellt werden, indem man ein Azetidinon der Formel

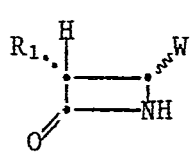

(IV)

durch Umsetzen mit einem Alkalimetallsalz, z.B. dem Natriumsalz, einer Thioniederalkancarbonsäure, z.B. Thioessigsäure, oder des Triphenylmethylmercaptans in eine Verbindung der Formel

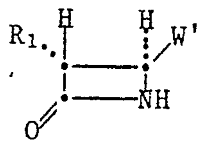

(VIII)

überführt, worin W' Triphenylmethylthio oder Niederalkanoylthio, z.B. Acetylthio, bedeutet, diese analog dem in den Reaktionsstufen 3 und 4 beschriebenen Verfahren in eine Verbindung der Formel

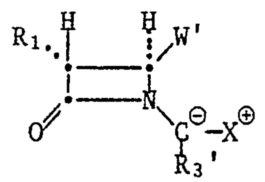

(IX)

überführt und diese in Gegenwart einer Base, z.B. Pyridin oder Tri-n-butylamin, in einem geeigneten Lösungsmittel, z.B. Diäthyläther oder Methanol, mit einem Salz der Formel MA, worin M die obige Bedeutung hat, insbesondere aber für ein Silber-Kation steht, und A ein gebräuchliches Anion darstellt, welches die Löslichkeit des Salzes MA in dem gewählten Lösungsmittel begünstigt, z.B. das Nitrat-, Acetat-oder Fluorid-Anion, umsetzt.

Die Ylide der Formel II können direkt in die Cyclisierungsreaktion zur Herstellung der Endprodukte der Formel I eingesetzt werden. Man kann aber auch in Verbindungen der Formel II, worin $R_1$ durch eine geschützte Hydroxygruppe, z.B. eine hydrolytisch leicht spaltbare geschützte Hydroxygruppe, wie trisubstituiertes Silyloxy, substituiertes Niederalkyl ist, zuerst die Hydroxyschutzgruppe abspalten und dann die erhaltene Verbindung der Formel II, worin $R_1$ durch Hydroxy substituiertes Niederalkyl ist, in die Cyclisierungsreaktion einsetzen.

In den Verbindungen der Formeln (II)-(VII) können vorhandene funktionelle Gruppen in geschützte funktionelle Gruppen oder vorhandene geschützte funktionelle Gruppen in die freien oder in anders geschützte Gruppen überführt werden. Weiterhin kann man in Verbindungen der Formeln (II), -(III), (V) und (VI) einen Rest $R_2$ in einen anderen Rest $R_2$ umwandeln. Bei diesen Umwandlungen können unter Berücksichtigung der weiteren in den Molekülen enthaltenen Substituenten die gleichen Methoden zur Anwendung gelangen, wie bei den entsprechenden Umwandlungen in die Verbindungen der Formel (I) angegeben ist.

Man kann das in Reaktionschema 1 beschriebene Verfahren zur Herstellung der Verbindungen der Formeln (II), (III) und (V)-(VII), sowie die angegebenen Verfahren zur Herstellung der Endprodukte der Formel (I) auch mit optisch inaktiven Verbindungen durchführen und auf einer beliebigen Verfahrensstufe aus einem erhaltenen Diastereomerengemisch oder Racemat, wie oben beschrieben, die optisch aktiven Verbindungen gemäss der vorliegenden Erfindung isolieren.

Die Erfindung betrifft ebenfalls die neuen Ausgangsverbindungen sowie verfahrensgemäss erhältliche neue Zwischenprodukte, wie diejenigen der Formeln (II), (III), (V) und (VI), sowie die angegebenen Verfahren zu ihrer Herstellung.

Vorzugsweise werden solche Ausgangsverbindungen verwendet und die Reaktionsbedingungen so gewählt, dass man zu den vorstehend als besonders bevorzugt aufgeführten Verbindungen gelangt.

Die Verbindungen der Formel I weisen wertvolle pharmakologische Eigenschaften auf oder können als Zwischenprodukte zur Herstellung von solchen Verbindungen mit wertvollen pharmakologischen Eigenschaften verwendet werden. Verbindungen der Formel I, worin $R_1$ durch Hydroxy substituiertes Niederalkyl ist, $R_2$ die unter Formel I angegebene Bedeutung hat und $R_3$ Carboxyl oder eine unter physiologischen Bedingungen spaltbare veresterte Carboxylgruppe darstellt und pharmakologisch annehmbare Salze von solchen Verbindungen mit salzbildenden Gruppen haben antibakterielle Wirkungen. Beispielsweise sind sie in vitro gegen grampositive und gramnegative Kokken, inkl. Enterokokken, z.B. Staphylococcus

aureus, Streptococcus pyogenes, Streptococcus pneumoniae und Streptococcus faecalis , und Neisseria sp. in minimalen Konzentrationen von ca. 0,005 bis ca. 4 µg/ml und gegen gramnegative Stäbchenbakterien, wie Enterobacteriaceae, Haemophilus influenzae und Pseudomonas aeruginosa, und Anaerobier, z.B. Bacteroides sp. , in minimale Konzentrationen von ca. 0,05 bis ca. 64 µg/ml wirksam. In vivo, bei der systemischen Infektion der Maus, z.B. durch Staphylococcus aureus, Streptococcus pyogenes und Escherichia coli, ergeben sich bei subkutaner Applikation erfindungsgemässer Verbindungen $ED_{50}$-Werte von ca. 0,8 bis ca. 30 mg/kg.

Die neuen Verbindungen können als oral oder parenteral applizierbare antibakterielle Antibiotika, z.B. in Form von entsprechenden pharmazeutischen Präparaten, zur Behandlung von Infektionen Verwendung finden.

Verbindungen der Formel I, worin mindestens eine der vorhandenen funktionellen Gruppen in geschützter Form vorliegt, können als Zwischenprodukte zur Herstellung der oben genannten pharmakologisch wirksamen Verbindungen der Formel I verwendet werden.

Die pharmakologisch verwendbaren Verbindungen der vorliegenden Erfindung können z.B. zur Herstellung von pharmazeutischen Präparaten verwendet werden, welche eine therapeutisch wirksame Menge der Aktivsubstanz zusammen oder im Gemisch mit anorganischen oder organischen, festen oder flüssigen, pharmazeutisch annehmbaren Trägerstoffen enthalten, die sich zur oralen oder zur parenteralen, d.h. intramuskulären, subcutanen oder intraperitonealen, Verabreichung eignen.

Zur oralen Verabreichung verwendet man Tabletten oder Gelatinekapseln, welche den Wirkstoff zusammen mit Verdünnungsmitteln, z.B. Lactose, Dextrose, Sucrose, Mannitol, Sorbitol, Cellulose und/oder Glycin, und Schmiermitteln, z.B. Kieselerde, Talk, Stearinsäure oder Salze davon, wie Magnesium-oder Calciumstearat, und/oder Polyäthylenglykol, aufweisen. Tabletten enthalten ebenfalls Bindemittel, z.B. Magnesiumaluminiumsilikat, Stärken, wie Mais-, Weizen-, Reis-oder Pfeilwurzstärke, Gelatine, Tragacant, Methylcellulose, Natriumcarboxymethylcellulose und/oder Polyvinylpyrrolidon, und, wenn erwünscht, Sprengmittel, z.B. Stärken, Agar, Alginsäure oder Salze davon, wie Natriumalginat, und/oder Brausemischungen oder Adsorptionsmittel, Farbstoffe, Geschmackstoffe oder Süssmittel.

Zur parenteralen Verabreichung eignen sich in erster Linie Infusionslösungen, vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit enthalten, vor Gebrauch hergestellt werden können. Solche Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten.

Die vorliegenden pharmazeutischen Präparate, die, wenn erwünscht, weitere pharmakologisch wertvolle Stoffe enthalten können, werden in an sich bekannter Weise, z.B. mittels konventionellen Mischungs-, Lösungs-oder Lyophilisierungsverfahren, hergestellt und enthalten von etwa 0,1% bis 100 %, insbesondere von etwa 1% bis etwa 50 %, Lyophilisate bis zu 100% des Aktivstoffs.

Je nach Art der Infektion und Zustand des infizierten Organismus verwendet man tägliche Dosen (oral oder parenteral) von etwa 100 mg bis etwa 2 g zur Behandlung von Warmblütern (Menschen und Tiere) von etwa 70 kg Gewicht.

Die folgenden Beispiele dienen zur Illustration der Erfindung. Temperaturen werden in Celsiusgraden angegeben.

Experimenteller Teil

Beispiel 1: (5R,6S)-6-tert.Butyldimethylsilyloxymethyl-2-(4-methyl-thiazol-5-yl)-2-penem-3-carbonsäureallylester

Eine Lösung von 2,15 g (2,94 mMol) 2-[3S,4R)-3-(tert.Butyl-dimethylsilyloxymethyl)-4-(4-methyl-thiazol-5-yl-carbonylthio)-2-oxo-azetidin-1-yl]-2-triphenylphosphoranylidenessigsäureallylester in 2,1 Liter Toluol wird während 22 Stunden unter Argon auf 114° erwärmt, anschliessend am Rotationsverdampfer eingeengt und der Rückstand an 50 g Silicagel (Toluol/Aethylacetat 3:1) chromatographiert.

DC: Silicagel (Toluol/Aethylacetat 3:1) $R_f$ = 0,37;

IR ($CH_2Cl_2$): 1790; 1715; 1590 $cm^{-1}$.

Das Ausgangsmaterial wird wie folgt hergestellt:

a) 4-Methyl-thiazol-5-carbonsäure

11,2 g (65,6 mMol) 4-Methyl-thiazol-5-carbonsäureäthylester werden in 224 ml Aethanol gelöst und mit 72 ml 1N NaOH bei Raumtemperatur während 6 Stunden versetzt. Dann wird mit Aethylacetat versetzt und mit konz. Phosphorsäure auf pH 2 eingestellt. Die wässerige Phase wird

zweimal mit Aethylacetat extrahiert und die organische Phase zweimal mit gesättigter Natriumchloridlösung gewaschen. Die Aethylacetat-Lösung wird über Natriumsulfat getrocknet und am Rotationsverdampfer eingeengt.

DC: Silicagel (Chloroform/Methanol/Wasser/Eisessig 70:40:10:0,5); $R_f$ = 0,54.

b) 4-Methyl-thiazol-5-carbonsäurechlorid

Eine Lösung von 11,5 g (80,4 mMol) 4-Methyl-thiazol-5-carbonsäure in 230 ml Toluol und 117 ml (1,6 Mol) Thionylchlorid wird während 4 $\frac{1}{2}$ Stunden auf 110° erwärmt. Das Rohprodukt wird am Rotationsverdampfer eingeengt, der Rückstand in Toluol gelöst und dann wiederum eingeengt. Den Rückstand löst man in Aethylacetat und wäscht diese Lösung mit kalter gesättigter Natriumhydrogencarbonatlösung. Die organische Phase wird über Natriumsulfat getrocknet und am Rotationsverdampfer eingeengt.

IR ($CH_2Cl_2$): 1745; 1480 $cm^{-1}$.

c) 2-[(3S,4R)-3-tert.Butyldimethylsilyloxymethyl-4-(4-methyl-thiazol-5-yl-carbonylthio)-2-oxo-azetidin-1-yl]-2-triphenylphosphoranylidenessigsäureallylester

1,77 g (10,9 mMol) 4-Methyl-thiazol-5-carbonsäurechlorid in 20 ml Methylenchlorid werden bei 0° unter Stickstoff mit 6,0 g (8,4 mMol) Silbersalz des 2-[(3S,4R)-3-tert.Butyldimethylsilyloxymethyl-4-mercapto-2-oxo-azetidin-1-yl]-2-triphenylphosphoranylidenessigsäureallylesters (Europäische Patentanmeldung Nr. 125207) versetzt und nach Zugabe von 1 ml Pyridin (innerhalb 45 Minuten) während einer Stunde bei Raumtemperatur gerührt. Die Reaktionslösung wird durch Hyflo-Supercel® filtriert und das Filtrat eingeengt. Der Rückstand wird in Aethylacetat aufgenommen und mit gesättigter Natrium-Bicarbonatlösung und zweimal mit gesättigter Kochsalzlösung gewaschen. Nach dem Trocknen der organischen Phase über Natriumsulfat und Abdampfen des Lösungsmittels am Rotationsverdampfer wird der Rückstand an 90 g Silicagel (System Toluol/Aethylacetat 3:1) gereinigt.

DC: Silicagel (Toluol/Aethylacetat 1:1) $R_f$ = 0,43;

IR ($CH_2Cl_2$): 1750; 1650; 1615 $cm^{-1}$.

Beispiel 2: (5R,6S)-2-(4-Methyl-thiazol-5-yl)-6-hydroxymethyl-2-penem-3-carbonsäureallylester

Eine Lösung von 187 mg (0,41 mMol) (5R,6S)-6-tert.Butyldimethylsilyloxymethyl-2-(4-methyl-thiazol-5-yl)-2-penem-3-carbonsäure-allylester in 5,3 ml Tetrahydrofuran wird bei 0° mit 0,19 ml - (3,48 mMol) Eisessig versetzt und dann auf -78° gekühlt. Nach Zugabe von 0,99 ml 1M Tetrabutylammoniumfluoridlösung in 9 ml Tetrahydrofuran lässt man das Reaktionsgemisch für 3 1/2 Stunden bei Raumtemperatur rühren. Dann wird mit Aethylacetat verdünnt und mit gesättigter Natriumhydrogencarbonatlösung und zweimal mit gesättigter Natriumchloridlösung gewaschen. Die organische Phase wird mit Natriumsulfat getrocknet und das Lösungsmittel am Rotationsverdampfer eingeengt. Der Rückstand wird mittels präparativer Schichtchromatographie gereinigt (Silicagel, Toluol/Aethylacetat 1:1). DC: Silicagel (Toluol/Aethylacetat 1:1) $R_f$ = 0,29;

IR ($CH_2Cl_2$): 1795; 1715 $cm^{-1}$.

Beispiel 3: (5R,6S)-2-(4-Methyl-thiazol-5-yl)-6-hydroxymethyl-2-penem-3-carbonsäure

450 mg (1,33 mMol) (5R,6S)-2-(4-Methyl-thiazol-5-yl)-6-hydroxymethyl-2-penem-3-carbonsäureallylester werden in 14,7 ml Tetrahydrofuran gelöst, bei Raumtemperatur mit 14,3 mg Palladiumtetrakistriphenylphosphin und 0,41 ml Tributylzinnhydrid versetzt und während 20 Minuten gerührt. Danach fügt man 0,16 ml Eisessig zu und rührt während 30 Minuten bei Raumtemperatur. Anschliessend wird am Rotationsverdampfer eingeengt. Der Rückstand wird in kaltem Aethylacetat aufgenommen und mit Natriumhydrogencarbonat bis pH 6,5 versetzt. Die Phasen werden getrennt und die wässerige Phase zweimal mit Aethylacetat gewaschen. Die vereinigten organischen Phasen werden einmal mit wenig Natriumhydrogencarbonatlösung gewaschen. Die vereinigten wässerigen Phasen werden mit 20%iger Phosphorsäure auf pH 3 gestellt, das Produkt mit Aethylacetat extrahiert, die organische Phase über Natriumsulfat getrocknet und das Lösungsmittel am Rotationsverdampfer abgezogen. Smp.: 150°;

IR (DMSO-$d_6$): 1785; 1690; 1515 $cm^{-1}$;

UV(Aethanol): $\lambda_{max}$ =330 nm ($\epsilon$=6100), 247 nm - ($\epsilon$=6850).

Beispiel 4: (5R,6S)-6-[(1'R)-1'-Allyloxycarbonyloxyäthyl-2-(4-methyl-thiazol-5-yl)-2-penem-3-carbonsäureallylester

Eine Lösung von 107 mg (0,149 mMol) 2-[-(3S,4R)-3-[(1'R)-1-Allyloxycarbonyloxyäthyl]-4-[(4-methyl-thiazol-5-yl)-carbonylthio]-2-oxo-azetidin-1-yl]-2-triphenylphosphoranylidenessigsäureallylester in 107 ml Toluol wird während 21 Stunden unter Argon auf 114° erwärmt, anschliessend am Rotationsverdampfer eingeengt und der Rückstand mittels präparativer Schichtchromatographie (Silicagel, Toluol/Aethylacetat 1:1) gereinigt.

DC: Silicagel (Toluol/Aethylacetat 1:1) $R_f$ = 0,56;

IR ($CH_2Cl_2$): 1795, 1715; 1590 $cm^{-1}$.

Das Ausgangsmaterial wird wie folgt hergestellt:

a) (3S,4R)-3-[(1'R)-1'-Allyloxycarbonyloxyäthyl]-4-triphenylmethylthio-azetidin-2-on

7,22 g Triphenylmethylmercaptan werden in 40 ml Methanol bei 0° suspendiert und über 10 Minuten portionsweise mit insgesamt 1,29 g einer 55%igen Natriumhydrid-Suspension in Oel versetzt. Anschliessend wird eine Emulsion von 6,95 g (3S,4R)-3-[(1'R)-1'-Allyloxycarbonyloxyäthyl]-4-tert.butylsulfonyl-azetidin-2-on (Europäische Patentanmeldung Nr. 126709) in 40 ml Aceton und 40 ml Wasser über 30 Minuten zugetropft. Nach 30 Minuten Rühren bei 0° und 1 Stunde bei Raumtemperatur wird das Reaktionsgemisch am Rotationsverdampfer eingeengt, mit Methylenchlorid versetzt, und die wässrige Phase wird abgetrennt. Die organische Lösung wird mit Sole gewaschen und über Natriumsulfat getrocknet. Nach Einengen wird die rohe Titelverbindung durch Chromatographie auf Silicagel (Laufmittel Toluol/Aethylacetat 2:3) gereinigt.

DC (Toluol-Aethylacetat 2:3): $R_f$ = 0,59;

IR (Methylenchlorid: 3390; 1760 $cm^{-1}$.

b) 2-[(3S,4R)-3-[(1'R)-1'-Allyloxycarbonyloxyäthyl]-4-triphenylmethylthio-2-oxo-azetidin-2-yl]-2-hydroxyessigsäureallylester

7,95 g (3S,4R)-3-[(1'R)-1'-Allyloxycarbonyloxyäthyl]-4-triphenylmethylthio-azetidin-2-on und 4,03 g Glyoxylsäure-allylester-äthylhemiacetal in 100 ml abs. Toluol werden mit 40 g Molekularsieb - (45Å) versetzt und während 94 Stunden bei 60° gerührt. Nach Abfiltrieren und Einengen am Rotationsverdampfer unter vermindertem Druck wird das Rohprodukt durch Chromatographie an Silicagel gereinigt (Laufmittel Toluol/Aethylacetat 4:1 DC (Silicagel, Toluol/Aethylacetat 4:1); $R_f$ = 0,22 und 0,16; IR ($CH_2Cl_2$): 3520, 1760, 1745 $cm^{-1}$.

c) 2-[(3S,4R)-3-[(1'R)-1'-Allyloxycarbonyloxyäthyl]-4-triphenylmethylthio-2-oxo-azetidin-1-yl]-2-triphenylphosporanylidenessigsäure-allylester

Zu einer Lösung von 1,0 g 2-[(3S,4R)-3-[(1'R)-1'-Allyloxycarbonyloxyäthyl]-4-triphenylmethylthio-2-oxo-azetidin-1-yl]-2-hydroxyessigsäure-allylester in 10 ml Tetrahydrofuran werden unter Rühren bei -15° nacheinander 182 µl Thionylchlorid und 206 µl Pyridin innert 5 Minuten zugegeben. Die weisse Suspension wird 1 Stunde bei -10° nachgerührt und über Hyflo filtriert. Nach dem Waschen des Rückstandes mit Toluol wird am Rotationsverdampfer eingeengt. Der Rückstand wird in 10 ml Dioxan gelöst, mit 624 mg Triphenylphosphin und 0,257 ml 2,6-Lutidin versetzt und während 46 Stunden bei 80° Badtemperatur gerührt. Das Gemisch wird über Hyflo-Supercel® filtriert und dieser Rückstand mit Toluol nachgewaschen. Die vereinigten Filtrate werden eingedampft. Die Chromatographie des Rückstandes an Silicagel ergibt das reine Produkt (Laufmittel Toluol/Aethylacetat 4:1); DC (Silicagel, Toluol-Aethylacetat 4:1): $R_f$ = 0,20; IR ($CH_2Cl_2$): 1745, 1605 $cm^{-1}$.

d) 2-[(3S,4R)-3-[(1'R)-1'-Allyloxycarbonyloxyäthyl]-4-mercapto-2-oxo-azetidin-1-yl]-2-triphenylphosphoranylidenessigsäure-allylester-Silbersalz

5,31 g 2-[(3S,4R)-3-[(1'R)-1'-Allyloxycarbonyloxyäthyl]-4-triphenylmethylthio-2-oxo-azetidin -1-yl]-2-triphenylphosphoranylidenessigsäure-allylester werden in 63 ml Aether vorgelegt und bei Raumtem peratur mit 50 ml einer 0,5 M wässrigen Silbernitrat-Lösung versetzt. Danach gibt man tropfenweise 14,7 ml eines Gemisches aus 3,6 ml Tributylamin, 0,18 ml Trifluoressigsäure und 25 ml Aether dazu und rührt das Reaktionsgemisch weitere 20 Minuten. Dann wird der Feststoff abgenutscht und mit Aether, Wasser and erneut mit Aether gewaschen . Der Feststoff wird zur Reinigung in 40 ml Aether und 40 ml Wasser aufgeschlämmt, abgenutscht und getrocknet. IR ($CH_2Cl_2$): 1760, 1620 $cm^{-1}$.

e) 2-[(3S,4R)-3-[(1'R)-1'-Allyloxycarbonyloxyäthyl]-4-[(4-methyl-thiazol-5-yl)-carbonylthio]-2-oxo-azetidin-1-yl]-2-triphenylphosphoranylidenessigsäureallylester

60,3 mg (0,37 mMol) 4-Methyl-thiazol-5-carbonsäurechlorid in 0,66 ml Methylenchlorid werden bei 0° unter Stickstoff mit 200 mg (0,298 mMol) Silbersalz (Beispiel 4d) versetzt und nach Zugabe von 35 µl Pyridin während 30 Minuten bei Raumtemperatur gerührt. Die Reaktionslösung wird eingeengt, der Rückstand in Aethylacetat aufgenommen, mit gesättigter Natriumhydrogencarbonatlösung und zweimal mit gesättigter Natriumchloridlösung gewaschen. Nach dem Trocknen der organischen Phase über Natriumsulfat und Abdampfen des Lösungsmittels am Rotationsverdampfer wird der Rückstand durch präparative Schichtchromatographie (Silicagel, Toluol/Aethylacetat 1:1) gereinigt.

DC: Silicagel (Toluol/Aethylacetat 1:1) $R_f$ = 0,38;

IR ($CH_2Cl_2$): 1750; 1650; 1620 $cm^{-1}$.

Beispiel 5: (5R,6S)-6-[(1'R)-1-Hydroxyäthyl]-2-(4-methyl-thiazol-5-yl)-2-penem-3-carbonsäure

239 mg (0,547 mMol) (5R,6S)-6-[(1'R)-1'-Allyloxycarbonyloxyäthyl]-2-(4-methyl-thiazol-5-yl)-2-penem-3-carbonsäureallylester werden in 6 ml Tetrahydrofuran gelöst und bei Raumtemperatur mit 12 mg Palladiumtetrakistriphenylphosphin und 0,34 ml Tetrabutylzinnhydrid versetzt und während 30 Minuten bei Raumtemperatur gerührt. Danach fügt man 67 µl (1,17 mMol) Eisessig zu und rührt während 50 Minuten. Anschliessend wird am Rotationsverdampfer eingeengt. Der Rückstand wird in kaltem Aethylacetat aufgenommen und mit Natriumhydrogen carbonat bis pH 6,5 versetzt. Die Phasen werden getrennt und die wässerige Phase zweimal mit Aethylacetat gewaschen. Die organischen Phasen werden mit wenig Natriumhydrogencarbonatlösung gewaschen. Die vereinigten wässerigen Phasen werden mit 20%iger Phosphorsäure auf pH 3 gestellt, das Produkt mit Aethylacetat extrahiert, die organische Phase über Natriumsulfat getrocknet und das Lösungsmittel am Rotationsverdampfer abgezogen. Der Rückstand wird mit Aether verrieben.

IR (DMSO-$d_6$): 1785; 1695; 1515 $cm^{-1}$;

UV (Aethanol): $\lambda_{max}$=330 nm ($\epsilon$=6200), 147 nm - ($\epsilon$=7250).

Beispiel 6: (5R,6S)-2-(2-Allyloxycarbonylamino-4-methyl-thiazol-5-yl)-6-tert.butyldimethylsilyloxymethyl-2-penem-3-carbonsäureallylester

Eine Lösung von 170 mg (0,2 mMol) 2-[-(3S,4R)-3-tert.Butyldimethylsilyloxymethyl-4-(2-allyloxycarbonylamino-4-methyl-thiazol-5-yl-carbonylthio]-2-oxo-azetidin-1-yl]-2-triphenylphosphoranylidenessigsäureallylester in 35 ml Toluol wird während 23 Stunden auf 115° erwärmt und anschliessend mit präparativer Schichtchromatographie (Silicagel, Toluol/Aethylacetat 2:1) gereinigt.

DC: Silicagel (Toluol/Aethylacetat 2:1) $R_f$ = 0,46;

IR ($CH_2Cl_2$): 3400; 1790; 1740 $cm^{-1}$.

Das Ausgangsmaterial wird wie folgt hergestellt:

a) 2-Allyloxycarbonylamino-4-methyl-thiazol-5-carbonsäureäthylester

Eine Lösung von 20,0 g (0,107 Mol) 2-Amino-4-methyl-thiazol-5-carbonsäureäthylester in 300 ml Tetrahydrofuran wird bei Raumtemperatur mit 26,0 ml Chlorameisensäureallylester versetzt und während ca. 45 Minuten werden 99 ml 2N Natronlauge zugetropft. Nach einer Stunde werden wiederum 26 ml Chlorameisensäureallylester und 99 ml 2N Natronlauge zugefügt. Nach 39 Stunden Reaktionszeit wird mit Aethylacetat verdünnt und diese Lösung zweimal mit Natriumchlorid lösung gewaschen. Die organischen Phasen werden über Natriumsulfat getrocknet und am Rotationsverdampfer eingeengt. Der Rückstand wird aus Tetrahydrofuran/Hexan umkristallisiert.

DC: Silicagel (Toluol/Aethylacetat 1:1) $R_f$ = 0,83;

IR ($CH_2Cl_2$): 3400; 1730; 1710; 1550 $cm^{-1}$.

b) 2-Allyloxycarbonylamino-4-methyl-thiazol-5-carbonsäure

Eine Lösung von 6,0 g (22,2 mMol) 2-Allyloxycarbonylamino-4-methyl-thiazol-5-carbonsäureäthylester in 60 ml Tetrahydrofuran wird mit 27,8 ml 4N Natriumhydroxid versetzt und während 20 Stunden bei 70° gerührt. Dann wird mit Aethylacetat verdünnt und die organische Phase mit gesättigter Natriumhydrogencarbonatlösung gewaschen. Die wässerigen Phasen werden auf 0° gekühlt und mit konz. Phosphorsäure auf pH 2 gestellt. Die ausgefallene Säure wird abfiltriert und

mit Wasser gewaschen. Der Rückstand wird am Hochvakuum getrocknet. Vor der Weiterverwendung werden die Kristalle zweimal mit Dimethylformamid am Hochvakuum eingedampft und getrocknet.

c) 2-Allyloxycarbonylamino-4-methyl-thiazol-5-thiocarbonsäure

Eine Lösung von 2,0 g (8,2 mMol) 2-Allyloxycarbonylamino-4-methyl-thiazol-5-carbonsäure in 34 ml Tetrahydrofuran und 4 ml Dimethylformamid wird mit 2,76 ml Triäthylamin bei Raumtemperatur während einer Stunde im Ultraschallbad gerührt. Dann wird die feine Suspension auf -50° gekühlt und mit 1,1 ml Chlorameisensäureäthylester (gelöst in 2 ml Tetrahydrofuran) versetzt. Nach einer Stunde bei -50° wird während 2 Stunden Schwefelwasserstoff eingeleitet. Die Lösung wird auf 0° erwärmt und mit 10 ml 2N Schwefelsäure versetzt. Die Thiosäure wird mit Aethylacetat extrahiert und zweimal mit gesättigter Natriumchloridlösung gewaschen. Die organische Phase wird über Natriumsulfat getrocknet und am Rotationsverdampfer eingeengt.

DC: Silicagel (Toluol/Aethylacetat/Eisessig 6:2:1) $R_f$ = 0,6;

IR (Dioxan): 3200; 1730; 1550 $cm^{-1}$.

d) (3S,4R)-4-(2-Allyloxycarbonylamino-4-methylthiazol-5-ylcarbonylthio)-3-tert.butyldimethylsilyloxymethyl-azetidin-2-on

342 mg (1,25 mMol) 2-Allyloxycarbonylamino-4-methyl-thiazol-5-thiocarbonsäure werden in 5,0 ml Aceton und 5 ml Wasser gelöst und bei Raumtemperatur unter Stickstoff mit Natriumhydrogencarbonat neutralisiert (bis pH 8). Nach 15 Minuten werden 295 mg (0,883 mMol) (3S,4R)-3-tert.Butyldimethylsilyloxymethyl-4-tert.butylsulfonyl-azetidin-2-on (Europäische Patentanmeldung Nr. 126709; gelöst in 5 ml Aceton) zugefügt und während 80 Minuten gerührt. Das Reaktionsgemisch wird mit Aethylacetat verdünnt und mit Natriumhydrogencarbonatlösung und gesättigter Natriumchloridlösung gewaschen. Die organische Phase wird mit Natriumsulfat getrocknet und am Rotationsverdampfer eingeengt. Das Produkt wird mittels präparativer Schichtchromatographie - (Toluol/Aethylacetat 2:1) gereinigt.

DC: Silicagel (Toluol/Aethylacetat 2:1) $R_f$ = 0,51;

IR ($CH_2Cl_2$): 3410; 3170; 1745; 1730 $cm^{-1}$.

e) 2-[(3S,4R)-3-tert.Butyldimethylsilyloxymethyl-4-(2-allyloxycarbonylamino-4-methyl-thiazol-5-ylcarbonylthio)-2-oxo-azetidin-1-yl]-2-hydroxyessigsäureallylester

Eine Lösung von 500 mg (1,06 mMol) (3S,4R)-4-(2-Allyloxycarbonylamino-4-methyl-thiazol-5-ylcarbonylthio)-3-tert.butyldimethylsilyloxymethyl-azetidin-2-on in 2,75 ml Toluol und 237 mg Glyoxylsäureallylester werden mit 500 mg Molekularsieb (4Å) bei Raumtemperatur während 20 Stunden gerührt. Anschliessend wird filtriert und das Toluol abgezogen. Das Produkt wird ohne weitere Reinigung weiterverwendet.

DC: Silicagel (Toluol/Aethylacetat 2:1) $R_f$ = 0,42;

IR ($CH_2Cl_2$): 3500; 3370; 1760j; 1725; 1660 $cm^{-1}$.

f) 2-[(3S,4R)-3-tert.Butyldimethylsilyloxymethyl-4-(2-allyloxycarbonylamino-4-methyl-thiazol-5-ylcarbonylthio)-2-oxo-azetidin-1-yl]-2-chloressigsäureallylester

Der rohe 2-[(3S,4R)-3-tert.Butyldimethylsilyloxymethyl-4-(2-allyloxycarbonylamino-4-methyl-thiazol-5-ylcarbonylthio)-2-oxo-azetidin-1-yl]-2-hydroxyessigsäureallylester (vgl. Beispiel 6e) wird in 3,6 ml Tetrahydrofuran gelöst, auf -15° gekühlt und dann mit 0,11 ml Pyridin und 92 µl Thionylchlorid versetzt. Nach 45 Minuten bei -15° wird die Suspension eingeengt und der Rückstand zweimal mit Toluol abgezogen. Das Rohprodukt wird ohne Reinigung weiterverwendet. DC: Silicagel - (Toluol/Aethylacetat 2:1) $R_f$ = 0,65;

IR ($CH_2Cl_2$): 3380; 1775; 1750; 1720; 1640 $cm^{-1}$.

g) 2-[(3S,4R)-3-tert.Butyldimethylsilyloxymethyl-4-(2-allyloxycarbonylamino-4-methyl-thiazol-5-ylcarbonylthio]-2-oxo-azetidin-1-yl]-2-triphenylphosphoranylidenessigsäureallylester

Der rohe 2-[(3S,4R)-3-tert.Butyldimethylsilyloxymethyl-4-(2-allyloxycarbonylamino-4-methyl-thiazol-5-ylcarbonylthio)-2-oxo-azetidin-1-yl]-2-chloressigsäureallylester (gelöst in 2,4 ml Dioxan) wird mit 369 mg Triphenylphosphin und 148 µl 2,6-Dimethylpyridin versetzt und während 24 Stunden auf 70° erwärmt. Dann wird mit Aethylacetat verdünnt und mit Natriumhydrogencarbonatlösung und zweimal mit Natriumchloridlösung gewaschen. Die organische Phase wird mit Natriumsulfat getrocknet und am Rotationsverdampfer eingeengt.

Das Produkt wird mit präparativer Schichtchromatographie (Toluol/Aethylacetat 2:1) gereinigt.

DC: Silicagel (Toluol/Aethylacetat 2:1) $R_f$ = 2,0;

IR ($CH_2Cl_2$): 3370; 1745; 1720; 1640; 1610 $cm^{-1}$.

Beispiel 7: (5R,6S)-2-(2-Allyloxycarbonylamino-4-methyl-thiazol-5-yl)-6-hydroxymethyl-2-penem-3-carbonsäureallylester

Eine Lösung von 174 mg (0,31 mMol) (5R,6S)-2-(2-Allyloxycarbonylamino-4-methylthiazol-5-yl)-6-tert.butyldimethylsilyloxymethyl-2-penem-3-carbonsäureallylester in 4 ml Tetrahydrofuran wird bei 0° mit 152 µl Eisessig versetzt und anschliessend auf -70° gekühlt. Zu dieser Lösung fügt man 0,76 ml (0,76 mMol) 1N Tetrabutylammoniumfluorid-Lösung in Tetrahydrofuran hinzu und lässt während 5 Stunden bei Raumtemperatur rühren. Nach Verdünnen mit Aethylacetat wäscht man die Lösung mit gesättigter Natriumhydrogencarbonatlösung und zweimal mit gesättigter Natriumchloridlösung. Die organische Phase wird mit Natriumsulfat getrocknet und am Rotationsverdampfer eingeengt. Das Produkt wird mit präparativer Schichtchromatographie (Silicagel, Toluol/Aethylacetat 1:2) gereinigt.

DC: Silicagel (Toluol/Aethylacetat 1:2) $R_f$ = 0,48;

IR ($CH_2Cl_2$): 3640; 3420; 3210; 1800; 1730 $cm^{-1}$.

Beispiel 8: Natrium (5R,6S)-2-(2-Amino-4-methyl-thiazol-5-yl)-6-hydroxymethyl-2-penem-3-carboxylat

Eine Lösung von 453 mg (1,03 mMol) (5R,6S)-2-(2-Allyloxycarbonylamino-4-methyl-thiazol-5-yl)-6-hydroxymethyl-2-penem-3-carbonsäureallylester in 9 ml Tetrahydrofuran wird während 5 Minuten mit Argon gespült und anschliessend mit 293 mg Dimedon und 120 mg Tetrakis-triphenylphosphinpalladium versetzt. Nach 1 3/4 Stunden bei Raumtemperatur wird mit Wasser versetzt und zweimal mit Aethylacetat gewaschen. Die organischen Phasen werden zweimal mit Wasser extrahiert. Die vereinigten wässerigen Phasen werden mit 0,1N Natriumhydroxid versetzt (bis pH 7) und die Lösung lyophilisiert. Der Rückstand wird an 10 g Opti $UPC_{12}$® (Laufmittel:Wasser) Silicagel chromatographiert und die das Produkt enthaltenden Fraktionen lyophilisiert.

DC: $UPC_{12}$ (Wasser) $R_f$ = 0,32;

IR (DMSO-$d_6$): 3300; 1770; 1620; 1520 $cm^{-1}$;

UV ($H_2O$): $\lambda_{max}$=335 nm ($\epsilon$=7480); 266 nm ($\epsilon$=7700).

Beispiel 9: (5R,6S)-2-(4-Allyloxycarbonylaminomethyl-thiazol-5-yl)-6-tert.butyldimethylsilyloxymethyl-2-penem-3-carbonsäureallylester

Eine Lösung von 100 mg (0,21 mMol) (3S,4R)-4-(4-Allyloxycarbonylaminomethyl-thiazol-5-ylcarbonylthio)-3-tert.butyldimethylsilyloxymethyl-azetidin-2-on in 2,9 ml Toluol wird auf -15° gekühlt und mit 35,5 µl (0,318 mMol) Allyloxyoxalylchlorid und 44 µl (0,318 mMol) Triäthylamin versetzt. Nach 1 Stunde bei -15° wird mit Toluol verdünnt und mit 0,1N Salzsäure, Wasser und gesättigter Natriumhydrogencarbonatlösung gewaschen. Die wässrigen Phasen werden zweimal mit Toluol gewaschen, die organischen Lösungen mit Natriumsulfat getrocknet und am Rotationsverdampfer eingeengt. Der Rück stand wird in 10 ml Toluol aufgenommen, die Lösung mit 84 µl Triäthylphosphit versetzt und während 5 3/4 Stunden auf 120° erwärmt. Das Produkt wird mit präparativer Schichtchromatographie (Silicagel, Toluol/Aethylacetat 2:1) gereinigt.

DC: Silicagel (Toluol/Aethylacetat 2:1) $R_f$ = 0,36;

IR ($CH_2Cl_2$: 3460; 1780; 1720 $cm^{-1}$.

Das Ausgangsmaterial wird wie folgt hergestellt:

a) 4-Brommethyl-thiazol-5-carbonsäureäthylester

Eine Lösung von 1,0 g (5,85 mMol) 4-Methyl-thiazol-5-carbonsäureäthylester in 12 ml Tetrachlorkohlenstoff wird mit 1,2 g (6,44 mMol) N-Bromsuccinimid und 21 mg Azoisobutyronitril während 30 Minuten mit UV-Licht bestrahlt. Nach Abkühlen der Suspension auf 0° wird vom Succinimid abfiltriert, das Filtrat am Rotationsverdampfer eingeengt und der Rückstand an 30 g Kieselgel (Toluol/Aethylacetat 20:1) chromatographisch gereinigt. Das Produkt zeigt folgende physikalische Daten:

DC: Silicagel (Toluol/Aetylacetat 6:1) $R_f$ = 0,56;

IR ($CH_2Cl_2$): 2980; 1720 $cm^{-1}$.

b) 4-Azidomethyl-thiazol-5-carbonsäureäthylester

Eine Lösung von 9,9 g (39,5 mMol) 4-Brommethyl-thiazol-5-carbonsäureäthylester in 100 ml Aceton wird mit 3,1 g (47,4 mMol) Natriumazid bei Raumtemperatur während 21 Stunden gerührt. Anschliessend wird mit Aethylacetat verdünnt und zweimal mit gesättigter Natriumchloridlösung gewaschen. Die organische Phase wird mit Natriumsulfat getrocknet und am Rotationsverdampfer eingeengt. Das Rohprodukt wird an 100 g Silicagel (Toluol/Aethylacetat 9:1) gereinigt.

DC: Toluol/Aethylacetat 6:1 $R_f$ = 0,5;

IR ($CH_2Cl_2$): 2980; 2100; 1715; 1520 $cm^{-1}$.

c) 4-Allyloxycarbonylaminomethyl-thiazol-5-carbonsäureäthylester

Eine Lösung von 750 mg (3,4 mMol) 4-Azidomethyl-thiazol-5-carbonsäureäthylester in 15 ml Tetrahydrofuran wird mit 375 mg Pd/C (10 %) versetzt und während 4 Stunden bei Raumtemperatur mit Wasserstoff hydriert. Der rohe 4-Aminomethyl-thiazol-5-carbonsäureäthylester wird für die nächste Reaktion ohne Reinigung weiterverwendet.

625 mg des rohen Amins werden in 9,7 ml Tetrahydrofuran gelöst und nach Zugabe von 0,56 ml (7,26 mMol) Chlorameisensäureallylester wird bei Raumtemperatur 2,55 ml 2N Natronlauge innert ca. 15 Minuten zugetropft. Nach 45 Minuten Rühren wird mit Aethylacetat verdünnt und mit gesättigter Natriumhydrogencarbonatlösung und Natriumchloridlösung gewaschen. Die organische Phase trocknet man mit Natriumsulfat und engt am Rotationsverdampfer ein. Der Rückstand wird in Aether gelöst und das Produkt mit Hexan gefällt.

DC: Silicagel (Toluol/Aethylacetat 2:1) $R_f$ = 0,3;

IR ($CH_2Cl_2$): 3440; 1720; 1500 $cm^{-1}$.

d) 4-Allyloxycarbonylaminomethyl-thiazol-5-carbonsäure

Eine Lösung von 6,9 g (25,6 mMol) 4-Allyloxycarbonylaminomethyl-thiazol-5-carbonsäureäthylester in 69 ml Tetrahydrofuran wird bei Raumtemperatur mit 32 ml 4N Natronlauge versetzt und während 6 Stunden gerührt. Anschliessend wird mit Aethylacetat verdünnt und mit gesättigter Natriumhydrogencarbonatlösung gewaschen. Die wässerige Phase wird mit Eis gekühlt und mit konz. Phosphorsäure auf pH 2 eingestellt. Die Carbonsäure wird mit Aethylacetat extrahiert und die organische Phase über Natriumsulfat getrocknet und am Rotationsverdampfer eingeengt.

DC: Silicagel (Toluol/Aethylacetat/Eisessig 6:2:1) $R_f$ = 0,16;

IR (Dioxan): 3360; 1740; 1540 $cm^{-1}$.

e) 4-Allyloxycarbonylaminomethyl-thiazol-5-thiocarbonsäure

Eine Lösung von 0,5 g (2,1 mMol) ±4-Allyloxycarbonylaminomethylthiazol-5-carbonsäure in 10 ml Tetrahydrofuran wird mit 0,69 ml Triäthylamin versetzt und auf -50° gekühlt. Dann werden 0,275 ml Chlorameisensäureäthylester zugefügt. Nach einer Stunde Reaktions dauer leitet man Schwefelwasserstoffgas ein. Nach zwei Stunden wird auf 0° erwärmt und mit 2,5 ml 2N Schwefelsäure versetzt. Die Thiosäure wird mit Aethylacetat extrahiert und die organische Phase mit Natriumsulfat getrocknet und am Rotationsverdampfer eingeengt. DC: Silicagel (Toluol/Aethylacetat/Eisessig 6:2:1) $R_f$ = 0,32;

IR ($CH_2Cl_2$): 3440; 1710; 1650; 1490 $cm^{-1}$.

f) (3S,4R)-4-(4-Allyloxycarbonylaminomethyl-thiazol-5-ylcarbonylthio)-3-tert.butyldimethylsilyloxymethyl-azetidin-2-on

560 mg (2,06 mMol) 4-Allyloxycarbonylaminomethyl-thiazol-5-thiocarbonsäure wird in einem Gemisch von 7,8 ml Aceton und 7,8 ml Wasser gelöst und mit gesättigter Natriumhydrogencarbonatlösung versetzt (bis pH 8). Dann fügt man eine Lösung von 526 mg (1,57 mMol) (3S,4R))-3-tert.Butyldimethylsilyloxymethyl-4-tert.butylsulfonyl-azetidin-2-on (Europäische Patentanmeldung Nr. 126709) in 7,8 ml Aceton zu und hält den pH der Lösung konstant auf etwa 9 durch Zufügen von 1N Natronlauge. Nach 23 Stunden bei Raumtemperatur wird mit Aethylacetat verdünnt und zweimal mit Natriumchloridlösung gewaschen. Die organische Phase wird mit Natriumsulfat getrocknet und am Rotationsverdampfer eingeengt. Das Produkt wird durch präparative Schichtchromatographie - (Silicagel, Toluol/Aethylacetat 2:1) gereinigt.

DC: Silicagel (Toluol/Aethylacetat 2:1) $R_f$ = 0,22;

IR ($CH_2Cl_2$): 3640; 3400; 1775; 1720; 1640 $cm^{-1}$.

Beispiel 10: (5R,6S)-2-(4-Allyloxycarbonylaminomethyl-thiazol-5-yl)-6-hydroxymethyl-2-penem-3-carbonsäureallylester

In analoger Weise wie in Beispiel 7 beschrieben werden 100 mg (5R,6S)-2-(4-Allyloxycarbonylaminomethyl-thiazol-5-yl)-6-tert.butyldimethylsilyloxymethyl-2-penem-3-carbonsäureallylester zu dem Titelprodukt umgesetzt.

IR ($CH_2Cl_2$): 3600; 3420; 1770; 1715; 1510 $cm^{-1}$.

Beispiel 11: (5R,6S)-2-(4-Aminomethyl-thiazol-5-yl)-6-hydroxymethyl-2-penem-3-carbonsäure

In analoger Weise wie in Beispiel 8 beschrieben werden 158 mg (5R,6S)-2-(4-Allyloxycarbonylaminomethyl-thiazol-5-yl)-6-hydroxymethyl-2-penem-3-carbonsäureallylester zu der Titelverbindung umgesetzt.

IR (DMSO-$d_6$): 1780; 1625; 1410 $cm^{-1}$;

$\alpha_D$ ($H_2O$, c = 0,269 %) = 92,9 ± 3,7°;

UV (Aethanol): $\lambda_{max}$ = 322 nm ($\epsilon$ = 3900), 245 nm ($\epsilon$ = 8000).

Beispiel 12: (5R,6S)-6-tert.Butyldimethylsilyloxymethyl-2-(5-methyl-thiazol-4-yl)-2-penem-3-carbonsäureallylester

Eine Lösung von 498 mg (0,68 mMol) 2-[-(3S,4R)-3-tert.Butyldimethylsilyloxymethyl-4-(5-methyl-thiazol-4-ylcarbonylthio)-2-oxo-azetidin-1-yl]-2-triphenylphosphoranylidenessigsäureallylester in 100 ml Toluol wird während 26 Stunden am Rückfluss erhitzt. Die Lösung wird eingeengt und der Rückstand durch präparative Schichtchromatographie gereinigt (Silicagel, Toluol/Aethylacetat 1:1). DC: Silicagel (Toluol/Aethylacetat 1:1) $R_f$ = 0,7;

IR ($CH_2Cl_2$): 1780; 1710 $cm^{-1}$.

a) 5-Methyl-thiazol-4-carbonsäuremethylester

Eine Lösung von 5,0 g (29 mMol) 2-Amino-5-methyl-thiazol-4-carbonsäuremethylester wird in 154 ml unterphosphoriger Säure (30 %) gelöst und bei -5° mit einer Lösung von 4,35 g (63,2 mMol) Natriumnitrit in 17,3 ml Wasser (unter Niveau) versetzt. Die Suspension wird während 1 Stunde bei 0° und anschliessend 2 ½ Stunden bei Raumtemperatur gerührt. Dann wird eine Lösung von 24,7 g Natronlauge in 250 ml Wasser bis pH 8 zugegeben und zweimal mit Aether extrahiert. Die organischen Phasen werden mit gesättigter Natriumchloridlösung gewaschen und mit Natriumsulfat getrocknet. Das Lösungsmittel wird am Rotationsverdampfer entfernt und der Rückstand an 70 g Silicagel (Toluol/Aethylacetat 2:1) gereinigt.

DC: Silicagel (Toluol/Aethylacetat 2:1) $R_f$ = 0,31;

IR ($CH_2Cl_2$): 1715; 1515; 1430 $cm^{-1}$.

b) 5-Methyl-thiazol-4-carbonsäure

4,0 g (25,4 mMol) 5-Methyl-thiazol-4-carbonsäuremethylester werden in 80 ml Aethanol gelöst und mit 28 ml 1N Natronlauge 5 Stunden bei Raumtemperatur gerührt. Die Reaktionslösung wird mit Aethylacetat überschichtet und mit konz. Phosphorsäure auf pH 2 gestellt. Die wässrige Phase wird zweimal mit Aethylacetat nachextrahiert. Die organischen Phasen werden mit gesättigter Natriumchloridlösung gewaschen und mit Natriumsulfat getrocknet. Das Lösungsmittel wird am Rotationsverdampfer entfernt.

IR ($CH_2Cl_2$): 3400; 1700; 1530 $cm^{-1}$.

c) 5-Methyl-thiazol-4-carbonsäurechlorid

550 mg (3,8 mMol) 5-Methyl-thiazol-4-carbonsäure werden in 11 ml Toluol mit 5,6 ml Thionylchlorid versetzt und während 3 Stunden am Rückfluss erwärmt. Dann wird das Lösungsmittel am Hochvakuum abgezogen, der Rückstand in Toluol aufgenommen und wiederum abgezogen. Der Rückstand wird in kaltem Aethylacetat aufgenommen und rasch mit kalter gesättigter Natriumhydrogencarbonatlösung und zweimal mit Natriumchloridlösung gewaschen. Die Aethylacetatphase wird über Natriumsulfat getrocknet und das Lösungsmittel am Rotationsverdampfer eingeengt.

IR ($CH_2Cl_2$): 1745; 1480; 1430 $cm^{-1}$.

d) 2-[(3S,4R)-3-tert.Butyldimethylsilyloxymethyl-4-(5-methyl-thiazol-4-ylcarbonylthio)-2-oxo-azetidin-1-yl]-2-triphenylphosphoranylidenessigsäureallylester

Eine Lösung von 1,0 g (1,4 mMol) Silbersalz des 2-[(3S,4R)-3-tert.Butyldimethylsilyloxy-4-mercapto-2-oxo-azetidin-1-yl]-2-triphenylphosphoranylidenessigsäureallylesters wird mit 0,17 ml Pyridin und 295 mg (1,87 mMol) 5-Methyl-thiazol-4-carbonsäurechlorid versetzt und 3 ½ Stunden bei Raumtemperatur gerührt. Das

Reaktionsgemisch wird durch Hyflo-Supercel® filtriert und mit kalter gesättigter Natriumhydrogencarbonatlösung gewaschen. Die organische Phase wird über Natriumsulfat getrocknet und eingeengt. Das Produkt wird an 25 g Silicagel - (Toluol/Aethylacetat 2:1) gereinigt.

DC: Silicagel (Toluol/Aethylacetat 1:1) $R_f$ = 0,54;

IR ($CH_2Cl_2$): 1750; 1655; 1610 $cm^{-1}$.

Beispiel 13: (5R,6S)-6-Hydroxymethyl-2-(5-methyl-thiazol-4-yl)-2-penem-3-carbonsäureallylester

210 mg (0,46 mMol) (5R,6S)-6-tert.Butyldimethylsilyloxymethyl-2-(5-methyl-thiazol-4-yl)-2-penem-3-carbonsäureallylester in 6 ml Tetrahydrofuran wird bei 0° mit 0,22 ml Eisessig versetzt und auf -78° gekühlt. Dann werden 1,12 ml Tetrabutylammoniumfluorid in 10 ml Tetrahydrofuran zugegeben, und die Reaktionslösung wird während 20 Stunden bei Raumtemperatur gerührt. Die Lösung wird eingeengt, der Rückstand in Aethylacetat aufgenommen und mit gesättigter Natriumhydrogencarbonatlösung und Natriumchloridlösung gewaschen. Die organische Phase wird über Natriumsulfat getrocknet und am Rotationsverdampfer eingeengt. Das Produkt wird mittels präparativer Schichtchromatographie gereinigt - (Toluol/Aethylacetat 1:1).

DC: Silicagel (Toluol/Aethylacetat 1:1) $R_f$ = 0,17;

IR ($CH_2Cl_2$): 3610; 1790; 1710; 1590 $cm^{-1}$.

Beispiel 14: (5R,6S)-6-Hydroxymethyl-2-(5-methyl-thiazol-4-yl)-2-penem-3-carbonsäure

Eine Lösung von 657 mg (1,94 mMol) (5R,6S)-6-Hydroxymethyl-2-(5-methyl-thiazol-4-yl)-2-penem-3-carbonsäureallylester in 21 ml Tetrahydrofuran wird mit 21 mg Tetrakistriphenylphosphinpalladium und 0,62 ml Tributylzinnhydrid versetzt und 35 Minuten bei Raumtemperatur gerührt. Nach 2 Stunden Reaktionsdauer wird mit 0,24 ml Eisessig versetzt und weitere 30 Minuten bei Raumtemperatur gerührt. Das Reaktionsgemisch wird mit Aethylacetat verdünnt und mit ges. Natriumhydrogencarbonatlösung auf pH 6,5 eingestellt. Die organische Phase wird noch zweimal mit Wasser gewaschen und die wässerigen Phasen zweimal mit Aethylacetat extrahiert. Die wässrigen Phasen werden lyophilisiert und der Rückstand an Amberlite® (170 ml) mit Wasser gereinigt. Die wässerige Lösung wird mit 2N Salzsäure auf pH 2 gestellt und das Produkt mit Aethylacetat extrahiert. Die organische Phase wird mit Natriumsulfat getrocknet und am Rotationsverdampfer eingeengt.

DC: $UPC_{12}$ (Acetonitril/Wasser 1:9) $R_f$ = 0,4;

IR (DMSO-$d_6$): 2930; 1785; 1700; 1590 $cm^{-1}$.

Beispiel 15: (5R,6S)-6-tert.Butyldimethylsilyloxymethyl-2-(2,4-dimethyl-thiazol-5-yl)-2-penem-3-carbonsäureallylester

Eine Lösung von 273 mg (0,36 mMol) 2-[-(3S,4R)-3-tert.Butyldimethylsilyloxymethyl-4-(2,4-dimethyl-thiazol-5-ylcarbonylthio)-2-oxo-azetidin-1-yl]-2-triphenylphosphoranylidenessigstureallylester in 200 ml Toluol wird während 22 Stunden unter Argon auf 114° erwärmt, anschliessend am Rotationsverdampfer eingeengt und der Rückstand an 10 g Silicagel (Toluol/Aethylacetat 3:1) chromatographiert.

DC: Silicagel (Toluol/Aethylacetat 1:1) $R_f$ = 0,52;

IR ($CH_2Cl_2$): 1790; 1715; 1590 $cm^{-1}$.

Das Ausgangsmaterial wird wie folgt hergestellt:

a) 2,4-Dimethyl-thiazol-5-carbonsäureäthylester

13,9 ml (0,1 Mol) 2-Chloracetessigsäureäthylester wird in 50 ml Aethanol mit 7,5 g (0,1 Mol) Thioacetamid versetzt und die Lösung während 16 Stunden bei 55° gerührt. Dann wird das Lösungsmittel am Rotationsverdampfer entfernt und der Rückstand in Aethylacetat aufgenommen. Die Lösung wird mit gesättigter Natriumhydrogencarbonlösung und zweimal mit wässeriger Natriumchloridlösung gewaschen. Die organische Phase wird mit Natriumsulfat getrocknet und am Rotationsverdampfer vollständig eingeengt. Das Produkt wird aus Wasser/Aethylalkohol umkristallisiert. Smp. 45-46°;

DC (Silicagel Toluol/Aethylacetat 1:1) $R_f$ = 0,59;

IR ($CH_2Cl_2$): 1710; 1535 $cm^{-1}$.

b) 2,4-Dimethyl-thiazol-5-carbonsäure

500 mg (2,7 mMol) 2,4-Dimethyl-thiazol-5-carbonsäureäthylester werden in 224 ml Aethanol gelöst und mit 3 ml 1N NaOH bei Raumtemperatur während 4 Stunden versetzt. Dann wird mit Aethylacetat versetzt und mit konz. Phosphorsäure auf pH 2 eingestellt. Die wässerige Phase wird zweimal mit Aethylacetat extrahiert und die organische Phase zweimal mit gesättigter Natriumchloridlösung gewaschen. Die Aethylacetat-Lösung wird über Natriumsulfat getrocknet und am Rotationsverdampfer eingeengt.

DC: Silicagel (Chloroform/Methanol/Wasser/Eisessig 70:40:10:0,5);

$R_f$ = 0,54.

c) 2,4-Dimethyl-thiazol-5-carbonsäurechlorid

Eine Lösung von 300 mg (1,9 mMol) 2,4-Dimethyl-thiazol-5-carbonsäure in 6 ml Toluol und 2,8 ml Thionylchlorid wird während 4 1/2 Stunden auf 110° erwärmt. Das Rohprodukt wird am Rotationsverdampfer eingeengt, der Rückstand in Toluol gelöst und dann wiederum eingeengt. Den Rückstand löst man in Aethylacetat und wäscht diese Lösung mit kalter gesättigter Natriumhydrogencarbonatlösung. Die organische Phase wird über Natriumsulfat getrocknet und am Rotationsverdampfer eingeengt.

IR ($CH_2Cl_2$): 1745; 1480 $cm^{-1}$.

d) 2,4-Dimethyl-thiazol-5-thiocarbonsäure

Eine Lösung von 500 mg (3,2 mMol) 2,4-Dimethyl-thiazol-5-carbonsäure in 8,2 ml Tetrahydrofuran wird mit 0,45 ml Triäthylamin bei -50° versetzt und mit 0,42 ml Chlorameisensäureäthylester (gelöst in 2 ml Tetrahydrofuran) versetzt. Nach einer Stunde bei -15°C wird während 2 Stunden Schwefelwasserstoff eingeleitet. Die Lösung wird auf 0° erwärmt und mit 10 ml 2N Schwefelsäure versetzt. Die Thiosäure wird mit Aethylacetat extrahiert und zweimal mit gesättigter Natriumchloridlösung gewaschen. Die organische Phase wird über Natriumsulfat getrocknet und am Rotationsverdampfer eingeengt.

DC: Silicagel (Aethylacetat/Eisessig 95:5) $R_f$ = 0,3.

e) (3S,4R)-4-(2,4-Dimethylthiazol-5-yl-carbonylthio)-3-tert.butyldimethylsilyloxymethyl-azetidin-2-on

2,1 g (12,1 mMol) 2,4-Dimethyl-thiazol-5-thiocarbonsäure werden in 42,0 ml Aceton gelöst und bei Raumtemperatur unter Stickstoff mit 12 ml 1 N Natronlauge neutralisiert (bis pH 8). Nach 15 Minuten werden 2,68 g (8,0 mMol) (3S,4R)-3-tert.Butyldimethylsilyloxymethyl-4-tert.butylsulfonyl-azetidin-2-on (gelöst in 5 ml Aceton) zugefügt und während 80 Minuten gerührt. Das Reaktionsgemisch wird mit Aethylacetat verdünnt und mit Natriumhydrogencarbonatlösung und gesättigter Natriumchloridlösung gewaschen. Die organische Phase wird mit Natriumsulfat getrocknet und am Rotationsverdampfer eingeengt. Das Produkt wird mittel präparativer Schichtchromatographie - (Toluol/Aethylacetat 1:1) gereinigt.

DC: Silicagel (Toluol/Aethylacetat 1:1) $R_f$ = 0,45;

IR ($CH_2Cl_2$): 3400; 1745 $cm^{-1}$

f) 2-[(3S,4R)-3-tert.Butyldimethylsilyloxymethyl-4-(2,4-dimethyl-thiazol-5-ylcarbonylthio)-2-oxo-azetidin-1-yl]-2-hydroxyessigsäureallylester

Eine Lösung von 300 mg (0,775 mMol) (3S,4R)-4-(2,4-Dimethyl-thiazol-5-ylcarbonylthio)-3-tert.butyldimethylsilyloxymethyl-azetidin-2-on in 3,0 ml Toluol und 211 mg Glyoxylsäureallylester werden mit 300 mg Molekularsieb (4Å) bei 60° während 9 Stunden gerührt. Anschliessend wird filtriert und das Toluol abgezogen. Das Produkt wird ohne weitere Reinigung weiterverwendet.

DC: Silicagel (Toluol/Aethylacetat 1:1) $R_f$ = 0,35;

IR ($CH_2Cl_2$): 3500; 3370; 1760; 1725 $cm^{-1}$.

g) 2-[(3S,4R)-3-tert.Butyldimethylsilyloxymethyl-4-(2,4-dimethyl-thiazol-5-ylcarbonylthio)-2-oxo-azetidin-1-yl]-2-chloressigsäureallylester

Der rohe 2-[(3S,4R)-3-tert.Butyldimethylsilyloxymethyl-4-(2-allyloxycarbonylamino-2,4-dimethyl-thiazol-5-ylcarbonylthio)-2-oxo-azetidin-1-yl]-2-hydroxyessigsäureallylester wird in 9,0 ml Tetrahydrofuran gelöst, auf -15° gekühlt und dann mit 0,08 ml Pyridin und 70 µl Thionylchlorid versetzt. Nach 40 Minuten bei -15° wird die Suspension eingeengt und der Rückstand zweimal mit Toluol abgezogen. Das Rohprodukt wird ohne Reinigung weiterverwendet.

DC: Silicagel (Toluol/Aethylacetat 1:1) $R_f$ = 0,67;

IR ($CH_2Cl_2$): 1775; 1750; 1720 $cm^{-1}$.

h) 2-[(3S,4R)-3-tert.Butyldimethylsilyloxymethyl-4-(2,4-dimethylthiazol-5-yl-carbonylthio)-2-oxo-azetidin-1-yl]-2-triphenylphosphoranylidenessigsäureallylester

Der rohe 2-[(3S,4R)-3-tert.Butyldimethylsilyloxymethyl-4-(2,4-dimethyl-thiazol-5-ylcarbonylthio)-2-oxo-azetidin-1-yl]-2-chloressigsäureallylester (gelöst in 8,0 ml Dioxan) wird mit 264 mg Triphenylphosphin und 110 µl 2,6-Dimethylpyridin versetzt und während 24 Stunden auf 70° erwärmt. Dann wird mit Aethylacetat verdünnt und mit Natriumhydrogencarbonatlösung und zweimal mit Natriumchloridlösung gewaschen. Die organische Phase wird mit Natriumsulfat getrocknet und am Rotationsverdampfer eingeengt. Das Produkt wird mit präparativer Schichtchromatographie (Toluol/Aethylacetat 1:2) gereinigt.

DC: Silicagel (Toluol/Aethylacetat 1:1) $R_f$ = 0,25;

IR ($CH_2Cl_2$): 1775; 1745; 1640; 1610 $cm^{-1}$.

Beispiel 16: (5R,6S)-2-(2,4-Dimethyl-thiazol-5-yl)-6-hydroxymethyl-2-penem-3-carbonsäureallylester

Eine Lösung von 288 mg (0,61 mMol) (5R,6S)-2-(2,4-Dimethylthiazol-5-yl)-6-tert.butyldimethylsilyloxymethyl-2-penem-3-carbonsäureallylester in 9,7 ml Tetrahydrofuran wird bei 0° mit 250 µl Eisessig versetzt und anschliessend auf -70° gekühlt. Zu dieser Lösung fügt man 1,35 ml (1,35 mMol) 1N Tetrabutylammoniumfluorid-Lösung in Tetrahydrofuran hinzu und lässt während 5 Stunden bei Raumtemperatur rühren. Nach Verdünnen mit Aethylacetat wäscht man die Lösung mit gesättigter Natriumhydrogencarbonatlösung und zweimal mit gesättigter Natriumchloridlösung. Die organische Phase wird mit Natriumsulfat getrocknet und am Rotationsverdampfer eingeengt. Das Produkt wird mit präparativer Schichtchromatographie (Silicagel, Toluol/Aethylacetat 1:2) gereinigt.

DC: Silicagel (Toluol/Aethylacetat 1:1) $R_f$ = 0,17;

IR ($CH_2Cl_2$): 3600; 3210; 1790; 1720 $cm^{-1}$.

Beispiel 17: Natrium-(5R,6S)-2-(2,4-Dimethyl-thiazol-5-yl)-6-hydroxymethyl-2-penem-3-carboxylat

Eine Lösung von 1,5 g (4,2 mMol) (5R,6S)-2-(2,4-Dimethyl-thiazol-5-yl)-6-hydroxymethyl-2-penem-3-carbonsäureallylester in 3,7 ml Tetrahydrofuran wird während 5 Minuten mit Argon gespült und anschliessend mit 600 mg Dimedon und 150 mg Tetrakis-triphenylphosphinpalladium versetzt. Nach 1 ¾ Stunden bei Raumtemperatur wird mit Wasser versetzt und zweimal mit Aethylacetat gewaschen. Die organischen Phasen werden zweimal mit Wasser extrahiert. Die vereinigten wässerigen Phasen werden mit 0,1N Natriumhydroxid versetzt (bis pH 7) und die Lösung lyophilisiert. Der Rückstand wird an 10 g Opti $UPC_{12}$ - (Laufmittel:Wasser) chromatographiert und die das Produkt enthaltenden Fraktionen lyophilisiert.

DC: $UPC_{12}$ (Wasser) $R_f$ = 0,15;

IR: (DMSO-$d_6$): 3400; 1750; 1620; $cm^{-1}$;

UV ($H_2O$): $\lambda_{max}$ = 258 nm ($\epsilon$ = 7300).

Beispiel 18: (5R,6S)-2-(Allyloxycarbonylaminomethyl-4-methyl-thiazol-5-yl)-6-tert.butyldimethylsilyloxymethyl-2-penem-3-carbonsäureallylester

Eine Lösung von 238 mg (0,49 mMol) (3S,4R)-4-(2-Allyloxycarbonylaminomethyl-4-methyl-thiazol-5-ylcarbonylthio)-3-tert.butyldimethylsilyloxymethyl-azetidin-2-on in 8 ml Methylenchlorid wird auf -15° gekühlt und mit 82 µl (0,735 mMol) Allyloxyoxalylchlorid und 126 µl (0,735 mMol) Hünigbase versetzt. Nach 1 Stunde bei -10° wird mit Toluol verdünnt und mit 0,1N Salzsäure, Wasser und gesättigter Natriumhydrogencarbonatlösung gewaschen. Die wässerigen Phasen werden zweimal mit Toluol gewaschen, die organischen Lösungen mit Natriumsulfat getrocknet und am Rotationsverdampfer eingeengt. Der Rückstand wird in 23 ml Toluol aufgenommen, die Lösung mit 190 µl Triäthylphosphit versetzt und während 16 Stunden auf 110° erwärmt. Das Produkt wird mit präparativer Schichtchromatographie (Silicagel, Toluol/Aethylacetat 2:1) gereinigt.

DC: Silicagel (Toluol/Aethylacetat 2:1) $R_f$ = 0,55;

IR ($CH_2Cl_2$): 3440; 1780; 1720 $cm^{-1}$.

Das Ausgangsmaterial wird wie folgt hergestellt:

a) 2-Brommethyl-4-methyl-thiazol-5-carbonsäureäthylester

Eine Lösung von 20,0 g (108 mMol) 2,4-Dimethyl-thiazol-5-carbonsäureäthylester in 200 ml Tetrachlorkohlenstoff wird mit 21,2 g (118,8 mMol) N-Bromsuccinimid und 400 mg Azobisisobutyronitril während 4 Stunden mit UV-Licht bestrahlt. Nach Abkühlen der Suspension auf 0° wird vom Succinimid abfiltriert, das Filtrat am Rotationsverdampfer eingeengt und der Rückstand an 400 g Kieselgel (Toluol/Aethylacetat 20:1) chromatographisch gereinigt. Das Produkt zeigt folgende physikalische Daten:

DC: Silicagel (Toluol/Aethylacetat 3:1) $R_f$ = 0,54;

IR ($CH_2Cl_2$): 2890; 1720 $cm^{-1}$.

b) 2-Azidomethyl-4-methyl-thiazol-5-carbonsäureäthylester

Eine Lösung von 0,5 g (1,9 mMol) 2-Brommethyl-4-methyl-thiazol-5-carbonsäureäthylester in 14 ml Aceton wird mit 0,3 g (4,7 mMol) Natriumazid bei Raumtemperatur während 30 Stunden gerührt. Anschliessend wird mit Aethylacetat verdünnt und zweimal mit gesättigter Natriumchloridlösung gewaschen. Die organische Phase wird mit Natriumsulfat getrocknet und am Rotationsverdampfer eingeengt. Das Rohprodukt wird an 4,0 g Silicagel (Toluol) gereinigt.

DC (Silicagel): Toluol/Aethylacetat 3:1 $R_f$ = 0,63;

IR ($CH_2Cl_2$): 2980; 2100; 1715; 1520 $cm^{-1}$.

c) 2-Allyloxycarbonylaminomethyl-4-methyl-thiazol-5-carbonsäureäthylester

Eine Lösung von 10,9 g (48,1 mMol) 2-Azidomethyl-4-methyl-thiazol-5-carbonsäureäthylester in 540 ml Tetrahydrofuran wird mit 5,4 g Pd/C (10 %) versetzt und während 5 Stunden bei Raumtemperatur mit Wasserstoff hydriert. Der rohe 2-Aminomethyl-4-methyl-thiazol-5-carbonsäureäthylester wird für die nächste Reaktion ohne Reinigung weiterverwendet.

7,8 g des rohen Amins werden in 5,9 ml Tetrahydrofuran gelöst und nach Zugabe von 8,3 ml (77,9 mMol) Chlorameisensäureallylester wird bei Raumtemperatur 20 ml gesättigter Natriumhydrogencarbonat-Lösung innert ca. 15 Minuten zugetropft. Nach 2 Stunden Rühren bei 0° wird mit Aethylacetat verdünnt und mit gesättigter Natriumhydrogencarbonatlösung und Natriumchloridlösung gewaschen. Die organische Phase trocknet man mit Natriumsulfat und engt am Rotationsverdampfer ein. Der Rückstand wird in Aether gelöst und das Produkt mit Hexan gefällt.

DC (Silicagel): Toluol/Aethylacetat (3:1) $R_f$ = 0,1;

IR ($CH_2Cl_2$): 3440; 1720; 1500 $cm^{-1}$.

d) 2-Allyloxycarbonylaminomethyl-4-methyl-thiazol-5-carbonsäure

Eine Lösung von 11,3 g (39,8 mMol) 2-Allyloxycarbonylaminomethyl-4-methyl-thiazol-5-carbonsäureäthylester in 110 ml Aethylalkohol wird bei Raumtemperatur mit 41 ml 1N Natronlauge versetzt und während 7 Stunden gerührt. Anschliessend wird mit Aethylacetat verdünnt und mit gesättigter Natriumhydrogencarbonatlösung gewaschen. Die wässerige Phase wird mit Eis gekühlt und mit konz. Phosphorsäure auf pH 2 eingestellt. Die Carbonsäure wird mit Aethylacetat extrahiert und die organische Phase über Natriumsulfat getrocknet und am Rotationsverdampfer eingeengt.

DC: Silicagel (Aethylacetat/Eisessig 95:5) $R_f$ = 0,57;

IR (Dioxan): 3360; 1740; 1540 $cm^{-1}$.

e) 2-Allyloxycarbonylaminomethyl-4-methyl-thiazol-5-thiocarbonsäure

Eine Lösung von 10 g (39 mMol) 2-Allyloxycarbonylaminomethyl-4-methyl-thiazol-5-carbonsäure in 100 ml Methylenchlorid wird mit 6,5 ml Triäthylamin versetzt und auf 0°C gekühlt. Dann werden 11,9 g Bis-(2-oxo-3-oxazolidinyl)-phosphinsäurechlorid zugefügt. Nach vier Stunden bei 0° wird 3,8 ml Pyridin zugegeben, und man leitet Schwefelwasserstoffgas ein. Nach zwei Stunden wird auf 0° erwärmt und mit 2,5 ml 2N Schwefelsäure versetzt. Die Thiosäure wird mit Chloroform extrahiert und die organische Phase mit Natriumsulfat getrocknet und am Rotationsverdampfer eingeengt.

DC: Silicagel (Aethylacetat/Eisessig 95:5) $R_f$ = 0,42;

IR ($CH_2Cl_2$): 3440; 1710; 1650; 1490 $cm^{-1}$.

f) (3S,4R)-4-(2-Allyloxycarbonylaminomethyl-4-methyl-thiazol-5-ylcarbonylthio)-3-tert.butyldimethylsilyloxymethyl-azetidin-2-on

2,25 g (8,2 mMol) 2-Allyloxycarbonylaminomethyl-4-methyl-thiazol-5-thiocarbonsäure wird in einem Gemisch von 3 ml Aceton und 7,8 ml Wasser gelöst und mit 8,2 ml 1N Natronlauge versetzt. Dann fügt man eine Lösung von 1,83 mg (5,47 mMol) (3S,4R)-3-(tert.Butyldimethylsilyloxymethyl)-4-tert.butylsulfonyl-azetidin-2-on (Europäische Patentanmeldung Nr. 126709) in 7,8 ml Aceton zu.

Nach 1 $\frac{1}{2}$ Stunden bei Raumtemperatur wird mit Aethylacetat verdünnt und zweimal mit Natriumchloridlösung gewaschen. Die organische Phase wird mit Natriumsulfat getrocknet und am Rotationsverdampfer eingeengt. Das Produkt wird durch präparative Schichtchromatographie (Silicagel, Toluol/Aethylacetat 2:1) gereinigt.

DC: Silicagel (Toluol/Aethylacetat 1:1) $R_f$ = 0,39,

IR ($CH_2Cl_2$): 3460; 3400; 1775; 1720; 1640 $cm^{-1}$.

Beispiel 19: (5R,6S)-2-(2-Allyloxycarbonylaminomethyl-4-methyl-thiazol-5-yl)-6-hydroxymethyl-2-penem-3-carbonsäureallylester

In analoger Weise wie in Beispiel 7 beschrieben werden 100 mg (5R,6S)-2-(2-Allyloxycarbonylaminomethyl-4-methyl-thiazol-5-yl)-6-(tert.butyldimethylsilyloxymethyl)-2-penem-3-carbonsäureallylester zu dem Titelprodukt umgesetzt.

DC: Silicagel (Toluol/Aethylacetat 1:1) $R_f$ = 0,16;

IR ($CH_2Cl_2$): 3600; 3440; 1790; 1720; 1510 $cm^{-1}$.

Beispiel 20: (5R,6S)-2-(2-Aminomethyl-4-methyl-thiazol-5-yl)-6-hydroxymethyl-2-penem-3-carbonsäure

In analoger Weise wie in Beispiel 8 beschrieben werden 158 mg (5R,6S)-2-(2-Allyloxycarbonylaminomethyl-4-methyl-thiazol-5-yl)-6-hydroxymethyl-2-penem-3-carbonsäureallylester zu der Titelverbindung umgesetzt.

DC: $UPC_{12}$ (Wasser/Acetonitril 4:1) $R_f$ = 0,48;

IR (DMSO-$d_6$): 3440; 1778; 1715; 1622 $cm^{-1}$.

Beispiel 21: (5R,6S)-2-(2-Allyloxycarbonylamino-5-methyl-thiazol-4-yl)-6-tert.butyldimethylsilyloxymethyl-2-penem-3-carbonsäureallylester

Eine Lösung von 434 mg (0,523 mMol) 2-[-(3S,4R)-3-tert.Butyl-dimethylsilyloxymethyl-4-(2-allyloxycarbonylamino-5-methyl-thiazol-4-yl-carbonylthio)-2-oxo-azetidin-1-yl]-2-triphenylphosphoranylidenessigsäureallylester in 90 ml Toluol wird während 22 Stunden auf 110° erwärmt und anschliessend mit präparativer Schichtchromatographie (Silicagel, Toluol/Aethylacetat 2:1) gereinigt.

DC: Silicagel (Toluol/Aethylacetat 2:1) $R_f$ = 0,67;

IR ($CH_2Cl_2$): 3400; 1790; 1740 $cm^{-1}$.

Das Ausgangsmaterial wird wie folgt hergestellt:

a) 2-Allyloxycarbonylamino-5-methyl-thiazol-4-carbonsäuremethylester

Eine Lösung von 15,7 g (89,4 mMol) 2-Amino-5-methyl-thiazol-4-carbonsäuremethylester in 257 ml Tetrahydrofuran wird bei Raumtemperatur mit 51,3 ml Chlorameisensäureallylester versetzt, und während ca. 45 Minuten werden 241 ml 2N Natronlauge zugetropft. Nach einer Stunde werden wiederum 9,7 ml Chlorameisensäureallylester und 44 ml 2N Natronlauge zugefügt. Nach 21 Stunden Reaktionszeit wird mit Aethylacetat verdünnt und diese Lösung zweimal mit Natriumchloridlösung gewaschen. Die organischen Phasen werden über Natriumsulfat getrocknet und am Rotationsverdampfer eingeengt. DC: Silicagel - (Toluol/Aethylacetat 3:1) $R_f$ = 0,19;

IR ($CH_2Cl_2$): 3430; 1730; 1710; 1550 $cm^{-1}$.

b) 2-Allyloxycarbonylamino-5-methyl-thiazol-4-carbonsäure

Eine Lösung von 15,7 g (61,5 mMol) 2-Allyloxycarbonylamino-5-methyl-thiazol-4-carbonsäuremethylester in 157 ml Tetrahydrofuran wird mit 123 ml 2N Natriumhydroxid versetzt und während 1 Stunde bei 70° gerührt. Dann wird mit Aethylacetat verdünnt und die organische Phase mit gesättigter Natriumhydrogencarbonatlösung gewaschen. Die wässerigen Phasen werden auf 0° gekühlt und mit konz. Phosphorsäure auf pH 2 gestellt. Die ausgefallene Säure wird abfiltriert und mit Wasser gewaschen. Der Rückstand wird am Hochvakuum getrocknet. Vor der Weiterverwendung werden die Kristalle zweimal mit Dimethylformamid am Hochvakuum eingedampft und getrocknet.

c) 2-Allyloxycarbonylamino-5-methyl-thiazol-4-thiocarbonsäure

Eine Lösung von 2,0 g (8,2 mMol) 2-Allyloxycarbonylamino-5-methyl-thiazol-4-carbonsäure in 20 ml Tetrahydrofuran wird mit 2,8 ml Triäthylamin bei Raumtemperatur während einer Stunde im Ultraschallbad gerührt. Dann wird die

feine Suspension auf -50° gekühlt und mit 1,1 ml Chlorameisensäureäthylester (gelöst in 2 ml Tetrahydrofuran) versetzt. Nach einer Stunde bei -50° wird während 2 Stunden Schwefelwasserstoff eingeleitet. Die Lösung wird auf 0° erwärmt und mit 10 ml 2N Schwefelsäure versetzt. Die Thiosäure wird mit Aethylacetat extrahiert und zweimal mit gesättigter Natriumchloridlösung gewaschen. Die organische Phase wird über Natriumsulfat getrocknet und am Rotationsverdampfer eingeengt.

DC: Silicagel (Toluol/Aethylacetat/Methanol 6:6:3) $R_f$ = 0,4;

IR (Dioxan): 3200; 1730; 1550 $cm^{-1}$.

d) (3S,4R)-4-(2-Allyloxycarbonylamino-5-methyl-thiazol-4-ylcarbonylthio)-3-tert.butyldimethylsilyloxymethyl-azetidin-2-on

1,7 g (6,6 mMol) 2-Allyloxycarbonylamino-5-methyl-thiazol-4-thiocarbonsäure werden in 25,0 ml Aceton und 25,0 Wasser gelöst und bei Raumtemperatur unter Stickstoff mit Natriumhydrogencarbonat neutralisiert (bis pH 8). Nach 15 Minuten werden 1,47 g (4,4 mMol) (3S,4R)-3-(tert.Butyldimethylsilyloxymethyl)-4-tert.butylsulfonyl-azetidin-2-on (Europäische Patentanmeldung Nr. 126709; gelöst in 5 ml Aceton) zugefügt und während 90 Minuten gerührt. Das Reaktionsgemisch wird mit Aethylacetat verdünnt und mit Natriumhydrogencarbonatlösung und gesättigter Natriumchloridlösung gewaschen. Die organische Phase wird mit Natriumsulfat getrocknet und am Rotationsverdampfer eingeengt. Das Produkt wird mittels präparativer Schichtchromatographie (Toluol/Aethylacetat 2:1) gereinigt.

DC: Silicagel (Toluol/Aethylacetat 2:1) $R_f$ = 0,57;

IR ($CH_2Cl_2$): 3410; 3170; 1775; 1730 $cm^{-1}$.

e) 2-[(3S,4R)-3-tert.Butyldimethylsilyloxymethyl-4-(2-allyloxycarbonylamino-5-methyl-thiazol-4-ylcarbonylthio)-2-oxo-azetidin-1-yl]-2-hydroxyessigsäureallylester

Eine Lösung von 500 mg (1,06 mMol) (3S,4R)-4-(2-Allyloxycarbonylamino-5-methyl-thiazol-4-ylcarbonylthio)-3-(tert.butyldimethylsilyloxymethyl)-azetidin-2-on in 2,75 ml Toluol und 240 mg Glyoxylsäureallylester werden mit 500 mg Molekularsieb (4Å) bei Raumtemperatur während 17 Stunden gerührt. Anschliessend wird filtriert und das Toluol abgezogen. Das Produkt wird ohne weitere Reinigung weiterverwendet.

DC: Silicagel (Toluol/Aethylacetat 2:1) $R_f$ = 0,51;

IR ($CH_2Cl_2$): 3500; 3370; 1760; 1725; 1660 $cm^{-1}$.

f) 2-[(3S,4R)-3-tert.Butyldimethylsilyloxymethyl-4-(2-allyloxycarbonylamino-5-methyl-thiazol-4-ylcarbonylthio)-2-oxo-azetidin-1-yl]-2-chloressigsäureallylester

Der rohe 2-[(3S,4R)-3-(tert.Butyldimethylsilyloxymethyl)-4-(2-allyloxycarbonylamino-5-methyl-thiazol-4-ylcarbonylthio)-2-oxo-azetidin-1-yl]-2-hydroxyessigsäureallylester wird in 3,6 ml Tetrahydrofuran gelöst, auf -15° gekühlt und dann mit 0,11 ml Pyridin und 94 µl Thionylchlorid versetzt. Nach 45 Minuten bei -15° wird die Suspension eingeengt und der Rückstand zweimal mit Toluol abgezogen. Das Rohprodukt wird ohne Reinigung weiterverwendet.

DC: Silicagel (Toluol/Aethylacetat 2:1) $R_f$ = 0,69;

IR ($CH_2Cl_2$): 3380; 1775; 1750; 1720; 1640 $cm^{-1}$.

g) 2-[(3S,4R)-3-tert.Butyldimethylsilyloxymethyl-4-(2-allyloxycarbonylamino-5-methyl-thiazol-4-yl-carbonylthio]-2-oxo-azetidin-1-yl]-2-triphenylphosphoranylidenessigsäureallylester

Der rohe 2-[(3S,4R)-3-tert.Butyldimethylsilyloxymethyl-4-(2-allyloxycarbonylamino-5-methyl-thiazol-4-ylcarbonylthio)-2-oxo-azetidin-1-yl]-2-chloressigsäureallylester, gelöst in 2,4 ml Dioxan, wird mit 365 mg Triphenylphosphin und 150 µl 2,6-Dimethylpyridin versetzt und während 21 Stunden auf 70° erwärmt. Dann wird mit Aethylacetat verdünnt und mit Natriumhydrogencarbonatlösung und zweimal mit Natriumchloridlösung gewaschen. Die organische Phase wird mit Natriumsulfat getrocknet und am Rotationsverdampfer eingeengt. Das Produkt wird mit präparativer Schichtchromatographie (Toluol/Aethylacetat 2:1) gereinigt.

DC: Silicagel (Toluol/Aethylacetat 2:1) $R_f$ = 0,37;

IR ($CH_2Cl_2$): 3370; 1745; 1720; 1640; 1610 $cm^{-1}$.

Beispiel 22: (5R,6S)-2-(2-Allyloxycarbonylamino-5-methyl-thiazol-4-yl)-6-hydroxymethyl-2-penem-3-carbonsäureallylester

Eine Lösung von 115 mg (0,21 mMol) (5R,6S)-2-(2-Allyloxycarbonylamino-5-methyl-thiazol-4-yl)-6-tert.butyldimethylsilyloxymethyl-2-penem-3-carbonsäureallylester in 3,3 ml Tetrahydrofuran wird bei 0° mit 100 µl Eisessig versetzt und anschliessend auf -70° gekühlt. Zu dieser Lösung fügt man 0,5 ml (0,5 mMol) 1N Tetrabutylammoniumfluorid-Lösung in Tetrahydrofuran hinzu und lässt während 5 Stunden bei Raumtemperatur rühren. Nach Verdünnen mit Aethylacetat wäscht man die Lösung mit gesättigter Natriumhydrogencarbonatlösung und zweimal mit gesättigter Natriumchloridlösung. Die organische Phase wird mit Natriumsulfat getrocknet und am Rotationsverdampfer eingeengt. Das Produkt wird mit präparativer Schichtchromatographie (Silicagel, Toluol/Aethylacetat 1:2) gereinigt.

DC: Silicagel (Toluol/Aethylacetat 1:2) $R_f$ = 0,42;

IR ($CH_2Cl_2$): 3640; 3420; 3210; 1800; 1730; $cm^{-1}$.

Beispiel 23: Natrium (5R,6S)-2-(2-Amino-5-methyl-thiazol-4-yl)-6-hydroxymethyl-2-penem-3-carboxylat

Eine Lösung von 1,5 g (3,42 mMol) (5R,6S)-2-(2-Allyloxycarbonylamino-5-methyl-thiazol-4-yl)-6-hydroxymethyl-2-penem-3-carbonsäureallylester in 30 ml Tetrahydrofuran wird während 5 Minuten mit Argon gespült und anschliessend mit 960 mg Dimedon und 398 mg Tetrakistriphenylphosphinpalladium versetzt. Nach 1 Stunde bei Raumtemperatur wird mit Wasser versetzt und zweimal mit Aethylacetat gewaschen. Die organischen Phasen werden zweimal mit Wasser extrahiert. Die vereinigten wässerigen Phasen werden mit 0,1N Natrium hydroxid versetzt (bis pH 7) und die Lösung lyophilisiert. Der Rückstand wird an 10 g Opti $UPC_{12}$ (Laufmittel:Wasser) chromatographiert und die das Produkt enthaltenden Fraktionen lyophilisiert. DC: $UPC_{12}$ (Wasser) $R_f$ = 0,41;

IR: (DMSO-$d_6$): 3300; 1770; 1620; 1520 $cm^{-1}$;

UV ($H_2O$): $\lambda_{max}$=335 nm ($\epsilon$=7480); 266 nm - ($\epsilon$=7700).

Beispiel 24: In analoger Weise, wie in den vorangehenden Beispielen beschrieben, werden die folgenden Verbindungen hergestellt:

(5R,6S)-2-(4-Methyl-oxazol-5-yl)-6-[(1R)-1-hydroxyäthyl]-2-penem-3-carbonsäure-natriumsalz,

$R_f$-Wert 0,57 ($UPC_{12}$-Platten; Wasser);

IR (Nujol): 1780; 1620; 1520 $cm^{-1}$; UV ($H_2O$): $\lambda_{max}$ = 324 nm.

Natrium-(5R,6S)-2-(5-amino-isothiazol-3-yl)-6-hydroxymethyl-2-penem-3-carboxylat,

$R_f$-Wert 0,70 ($UPC_{12}$-Platten; Wasser/Acetonitril 9:1);

IR (Nujol): 1770; 1615 $cm^{-1}$.

(5R,6S)-2-(4-Hydroxymethyl-thiazol-5-yl)-6-hydroxymethyl-2-penem-3-carbonsäure,

IR (DMSO-$d_6$): 3300; 1775; 1610; 1540 $cm^{-1}$.

(5R,6S)-2-(4-Brommethyl-thiazol-5-yl)-6-hydroxymethyl-2-penem-3-carbonsäure,

IR (DMSO-$d_6$): 3400; 1750; 1620 $cm^{-1}$.

(5R,6S)-2-(5-Methyl-isoxazol-4-yl)-6-hydroxymethyl-2-penem-3-carbonsäure-natriumsalz,

IR (DMSO-$d_6$): 1767 $cm^{-1}$; UV ($H_2O$) $\lambda_{max}$ = 3.13 nm.

(5R,6S)-2-(5-Methyl-isoxazol-3-yl)-6-hydroxymethyl-2-penem-3-carbonsäure-natriumsalz,

IR (DMSO-$d_6$): 1775; 1626 1602 $cm^{-1}$; UV ($H_2O$) $\lambda_{max}$ = 330 nm.

(5R,6S)-2-(5-Methyl-oxazol-4-yl)-6-[(1R)-1-hydroxyäthyl]-2-penem-3-carbonsäure-natriumsalz,

IR (DMSO-$d_6$): 3300; 1780; 1620 $cm^{-1}$; DC ($UPC_{12}$, Wasser): $R_f$ = 0,53.

Beispiel 25: (5R,6S)-6-Hydroxymethyl-2-(4-methyl-thiazol-5-yl)-2-penem-3-carbonsäure-acetoxymethylester

78 mg (0,26 mMol) (5R,6S)-6-Hydroxymethyl-2-(4-methyl-thiazol-5-yl)-2-penem-3-carbonsäure werden in 1 ml abs. DMF und 0,5 ml abs. DMSO gelöst und bei 0° unter Rühren mit 26,2 mg Kaliumhydrogencarbonat und 90 mg 18-Crown-6 und tropfenweise mit einer Lösung von 39,8 mg Acetoxybrommethan in 0,3 ml abs. DMF versetzt. Nach 30 Minuten Rühren bei Raumtemperatur wird das Reaktionsgemisch mit Aethylacetat verdünnt und zweimal mit Sole gewaschen. Die organische Phase wird über Magnesiumsulfat getrocknet und eingedampft. Die Reinigung durch Säulenchromatographie (Laufmittel Toluol/Aethylacetat 2:1) ergibt die Titelverbindung.

DC (Silicagel, Toluol/Aethylacetat 2:1) $R_f$= 0,07;

IR (Methylenchlorid): 3590; 1790; 1765; 1725; 1580 $cm^{-1}$.

Beispiel 26: (5R,6S)-6-Hydroxymethyl-2-(4-methyl-thiazol-5-yl)-2-penem-3-carbonsäure-1-äthoxycarbonyloxyäthylester

1,2 g Natriumjodid werden in 3,7 ml Aceton gelöst und mit 0,275 ml Aethyl-1-chloräthylcarbonat versetzt. Das Gemisch wird bei Raumtemperatur während 3 Stunden gerührt. Anschliessend wird die Lösung auf 15 ml Methylenchlorid getropft und von den ausgefallenen anorganischen Salzen abfiltriert. Die Methylenchloridlösung wird bis auf 2 ml eingeengt und bei 0° zu einer Lösung von 0,298 g (1 mMol) (5R,6S)-6-Hydroxymethyl-2-(4-methyl-thiazol-5-yl)-2-penem-3-carbonsäure in 4 ml Dimethylacetamid gegeben. Dann wird während 3 Stunden bei 0° gerührt, anschliessend mit Aethylacetat verdünnt und dreimal mit Wasser gewaschen. Die organischen Phasen werden über Natriumsulfat getrocknet und am Rotationsverdampfer eingeengt. Das Rohprodukt wird an 10 g Silicagel mit dem Laufmittel Aethylacetat gereinigt. Man erhält die Titelverbindung als weissen Schaum. IR-Spektrum (Methylenchlorid): 3600; 1790; 1750; 1720; 1670; $cm^{-1}$.

Beispiel 27: (5R,6S)-6-Hydroxymethyl-2-(4-methyl-thiazol-5-yl)-2-penem-3-carbonsäure-pivaloyloxymethylester

0,6 g Natriumjodid werden in 2 ml Aceton gelöst und mit 0,15 ml Pivalinsäurechlormethylester versetzt. Das Gemisch wird bei Raumtemperatur während 3 Stunden gerührt und anschliessend auf 7,5 ml Methylenchlorid getropft. Die ausgefallenen anorganischen Salze werden abfiltriert. Die Methylenchloridlösung wird bis auf 1 ml eingeengt und zu einer Lösung von 0,119 g (0,4 mMol (5R,6S)-6-Hydroxymethyl-2-(4-methyl-thiazol-5-yl)-2-penem-3-carbonsäure und 0,17 ml Diisopropyläthylamin in 4 ml N,N-Dimethylacetamid bei 0° gegeben. Dann wird während 3 Stunden bei 0° gerührt, anschliessend mit Aethylacetat verdünnt und dreimal mit Wasser gewaschen. Die organische Phase wird über Natriumsulfat getrocknet und am Rotationsverdampfer eingeengt. Das Rohprodukt wird an 10 g Silicagel mit dem Laufmittel Aethylacetat gereinigt. Man erhält die Titelverbindung als weissen Schaum. IR-Spektrum (Methylenchlorid): 3600; 1795; 1745; 1725; 1670 $cm^{-1}$.

Beispiel 28: Trockenampullen oder Vials, enthaltend 0,5 g (5R,6S)-2-(4-Methyl-thiazol-5-yl)-6-[-(1'R)-1'-hydroxyäthyl]-2-penem-3-carbonsäure als Wirksubstanz, werden wie folgt hergestellt:

Zusammensetzung (für 1 Ampulle oder Vial):

Wirksubstanz 0,5 g

Mannit 0,5 g

Eine sterile wässerige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

Anstelle des obengenannten Wirkstoffs kann auch dieselbe Menge eines anderen Wirkstoffs der vorangehenden Beispiele verwendet werden.

**Ansprüche**

1. Verbindungen der Formel

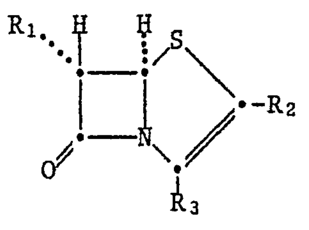

(I),

worin $R_1$ durch Hydroxy oder geschütztes Hydroxy substituiertes Niederalkyl ist, $R_2$ einen über ein Ringkohlenstoffatom an den Penem-Rest gebundenen monocyclischen 5-gliedrigen Heteroarylrest mit einem oder zwei Ringstickstoffatomen oder mit einem Ringstickstoffatom und einem zusätzlichen Ringsauerstoff-oder Ringschwefelatom darstellt, und $R_3$ Carboxyl oder funktionell abgewandeltes Carboxyl ist, und Salze von solchen Verbindungen der Formel I, die eine salzbildende Gruppe aufweisen.

2. Verbindungen der Formel I gemäss Anspruch 1, worin $R_1$ durch Hydroxy oder geschütztes Hydroxy substituiertes Niederalkyl ist, $R_2$ einen über ein Ringkohlenstoffatom an den Penem-Rest gebundenen aza-, diaza-, oxaza-oder thiaza-cyclischen Rest aromatischen Charakters darstellt, unsubstituiert

oder der zumindest an einem Ringkohlenstoffatom und gegebenenfalls zusätzlich an einem Ringstickstoffatom durch Hydroxy, Niederalkoxy, Niederalkanoyloxy, Halogen, Mercapto, Niederalkylthio, Phenylthio, Niederalkyl, Hydroxyniederalkyl, Niederalkanoyloxy-niederalkyl, Carboxyniederalkyl, Niederalkoxycarbonylniederalkyl, Carbamoylniederalkyl, Carbamoyloxyniederalkyl, Halogenniederalkyl, gegebenenfalls N-niederalkyliertes Aminoniederalkyl, Niederalkanoylaminoniederalkyl, Amino, Niederalkylamino, Diniederalkylamino, Niederalkanoylamino, Carboxyl Niederalkoxycarbonyl, Carbamoyl, N-mono-oder N,N-dinie deralkyliertes Carbamoyl, Cyano, Niederalkanoyl, gegebenenfalls durch Niederalkyl, Nitro, Niederalkoxy und/oder Halogen substituiertes Phenyl und/oder Nitro substituiert ist, und $R_3$ Carboxyl, unter physiologischen Bedingungen spaltbares verestertes Carboxyl oder geschütztes Carboxyl $R_3'$ bedeutet, und Salze von Verbindungen der Formel I, die eine salzbildende Gruppe aufweisen.

3. Verbindungen der Formel I gemäss Anspruch 1, worin $R_1$ durch Hydroxy substituiertes Niederalkyl ist, $R_2$ über ein Ringkohlenstoffatom an den Penem-Rest gebundenes unsubstituiertes oder durch Amino, Niederalkyl und/oder Aminoniederalkyl substituiertes Imidazolyl oder Pyrazolyl, unsubstituiertes oder durch Niederalkyl und/oder Niederalkoxy substituiertes Oxazolyl, unsubstituiertes oder durch Amino, Niederalkylamino, Diniederalkylamino, Niederalkanoylamino, Niederalkyl, Aminoniederalkyl, Hydroxyniederalkyl, Halogenniederalkyl, Carbamoyloxyniederalkyl und/oder Halogen substituiertes Thiazolyl, oder unsubstituiertes oder durch Niederalkyl, substituiertes Isothiazolyl, darstellt, und $R_3$ Carboxyl oder unter physiologischen Bedingungen spaltbares verestertes Carboxyl bedeutet, und Salze von Verbindungen der Formel I, die eine salzbildende Gruppe aufweisen.

4. Verbindungen der Formel I gemäss Anspruch 1, worin $R_1$ in $\alpha$-Stellung durch Hydroxy substituiertes Niederalkyl bedeutet, $R_2$ durch Amino und/oder Niederalkyl substituiertes Imidazol-4-yl, durch Amino, Niederalkyl, Hydroxyniederalkyl, Halogenniederalkyl und/oder Aminoniederalkyl substituiertes Thiazol-4-yl oder -5-yl, oder durch Amino substituiertes Isothiazol-4-yl darstellt, und $R_3$ Carboxyl oder unter physiologischen Bedingungen spaltbares verestertes Carboxyl ist, und Salze von Verbindungen der Formel I, die eine salzbildende Gruppe aufweisen.

5. Verbindungen der Formel I gemäss Anspruch 1, worin $R_1$ Hydroxymethyl oder 1-Hydroxyäthyl ist, $R_2$ an einem Ringkohlenstoffatom durch Niederalkyl, Amino und/oder Aminoniederalkyl substituiertes Thiazol-4-yl oder Thiazol-5-yl bedeutet und $R_3$ Carboxyl oder unter physiologischen Bedingungen spaltbares verestertes Carboxyl bedeutet, und Salze von solchen Verbindungen, welche eine salzbildende Gruppe aufweisen.

6. Pharmazeutisch annehmbare Salze von Verbindungen der Formel I gemäss Anspruch 1.

7. Unter physiologischen Bedingungen spaltbare Ester von Verbindungen der Formel I gemäss Anspruch 1.

8. (5R,6S)-2-(4-Methyl-thiazol-5-yl)-6-hydroxymethyl-2-penem-3-carbonsäure und pharmazeutisch annehmbare Salze davon gemäss Anspruch 1.

9. (5R,6S)-6-[(1'R)-1'-Hydroxyäthyl]-2-(4-methylthiazol-5-yl)-2-penem-3-carbonsäure und pharmazeutisch annehmbare Salze davon gemäss Anspruch 1.

10. (5R,6S)-2-(2-Amino-4-methyl-thiazol-5-yl)-6-hydroxymethyl-2-penem-3-carbonsäure und pharmazeutisch annehmbare Salze davon gemäss Anspruch 1.

11. (5R,6S)-6-Hydroxymethyl-2-(5-methyl-thiazol-4-yl)-2-penem-3-carbonsäure und pharmazeutisch annehmbare Salze davon gemäss Anspruch 1.

12. (5R,6S)-2-(2-Amino-5-methyl-thiazol-4-yl)-6-hydroxymethyl-2-penem-3-carbonsäure und pharmazeutisch annehmbare Salze davon gemäss Anspruch 1.

13. Pharmazeutisches Präparat enthaltend eine Verbindung der Formel I gemäss Anspruch 1 oder ein pharmazeutisch annehmbares Salz einer solchen Verbindung.

14. Verwendung von Verbindungen der Formel I gemäss Anspruch 1 und von pharmazeutisch annehmbaren Salzen solcher Verbindungen zur Herstellung von pharmazeutischen Präparaten.

15. Eine Verbindung der Formel I gemäss Anspruch 1 und pharmazeutisch annehmbare Salze solcher Verbindungen als antibiotische Mittel.

16. Eine Verbindung der Formel I gemäss Anspruch 1 und pharmazeutisch annehmbare Salze solcher Verbindungen zur Anwendung in einem

Verfahren zur therapeutischen Behandlung des menschlichen oder tierischen Körpers.

17. Verfahren zur Herstellung von Verbindungen der Formel I gemäss Anspruch 1, dadurch gekennzeichnet, dass man

a. eine Ylid-Verbindung der Formel

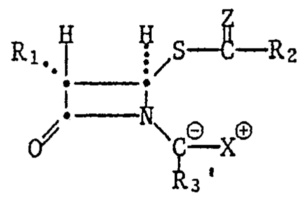

(II),

worin $R_1$ und $R_2$ die unter Formel I angegebenen Bedeutungen haben, $R_3$' eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b. eine Verbindung der Formel

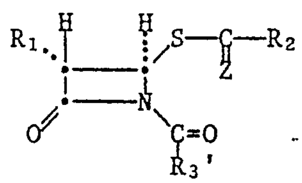

(III),

worin $R_1$ und $R_2$ die unter Formel I angegebenen Bedeutungen haben, Z die unter Formel II angegebene Bedeutung hat und $R_3$' eine geschützte Carboxylgruppe darstellt, mit einer organischen Verbindung des dreiwertigen Phosphors behandelt, und, wenn erwünscht oder notwendig, in einer erhältlichen Verbindung der Formel I eine geschützte funktionelle Gruppe in die freie funktionelle Gruppe überführt, und/oder, wenn erwünscht, in einer erhältlichen Verbindung der Formel I eine Carboxylgruppe $R_3$ in eine unter physiologischen Bedingungen spaltbare veresterte Carboxylgruppe überführt, und/oder, wenn erwünscht in einer erhältlichen Verbindung der Formel I einen Rest $R_2$ in einen anderen Rest $R_2$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

18. Die nach den Verfahren gemäss Anspruch 17 erhältlichen Verbindungen.

Patentansprüche für den Vertragsstaat : AT

1. Verfahren zur Herstellung von Verbindungen der Formel

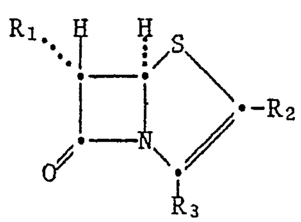

(I),

worin $R_1$ durch Hydroxy oder geschütztes Hydroxy substituiertes Niederalkyl ist, $R_2$ einen über ein Ringkohlenstoffatom an den Penem-Rest gebundenen monocyclischen 5-gliedrigen Heteroarylrest mit einem oder zwei Ringstickstoffatomen oder mit einem Ringstickstoffatom und einem zusätzlichen

Ringsauerstoff-oder Ringschwefelatom darstellt, und $R_3$ Carboxyl oder funktionell abgewandeltes Carboxyl ist, und Salzen von solchen Verbindungen der Formel I, die eine salzbildende Gruppe aufweisen, dadurch gekennzeichnet, dass man

a. eine Ylid-Verbindung der Formel

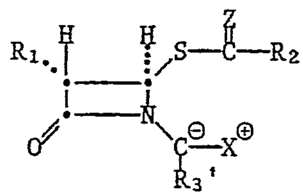

(II),

worin $R_1$ und $R_2$ die unter Formel I angegebenen Bedeutungen haben, $R_3'$ eine geschützte Carboxylgruppe darstellt, Z Sauerstoff oder Schwefel bedeutet und X⊕ entweder eine dreifach substituierte Phosphoniogruppe oder eine zweifach veresterte Phosphonogruppe zusammen mit einem Kation darstellt, ringschliesst, oder

b. eine Verbindung der Formel

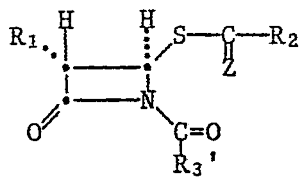

(III),

worin $R_1$ und $R_2$ die unter Formel I angegebenen Bedeutungen haben, Z die unter Formel II angegebene Bedeutung hat und $R_3'$ eine geschützte Carboxylgruppe darstellt, mit einer organischen Verbindung des dreiwertigen Phosphors behandelt, und, wenn erwünscht oder notwendig, in einer erhältlichen Verbindung der Formel I eine geschützte funktionelle Gruppe in die freie funktionelle Gruppe überführt, und/oder, wenn erwünscht, in einer erhältlichen Verbindung der Formel I eine Carboxylgruppe $R_3$ in eine unter physiologischen Bedingungen spaltbare veresterte Carboxylgruppe überführt, und/oder, wenn erwünscht in einer erhältlichen Verbindung der Formel I einen Rest $R_2$ in einen anderen Rest $R_2$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

2. Verfahren zur Herstellung von Verbindungen der Formel I gemäss Anspruch 1, worin $R_1$ Hydroxy oder geschütztes Hydroxy substituiertes Niederalkyl ist, $R_2$ einen über ein Ringkohlenstoffatom an den Penem-Rest gebundenen aza-, diaza-, oxaza-oder thiazacyclischen Rest aromatischen Charakters darstellt, unsubstituiert oder der zumindest an einem Ringkohlenstoffatom und gegebenenfalls zusätzlich an einem Ringstickstoffatom durch Hydroxy, Niederalkoxy, Niederalkanoyloxy, Halogen, Mercapto, Niederalkylthio, Phenylthio, Niederalkyl, Hydroxyniederalkyl, Niederalkanoyloxy-niederalkyl, Carboxyniederalkyl, Niederalkoxycarbonylniederalkyl, Carbamoylniederalkyl, Carbamoyloxyniederalkyl, Halogenniederalkyl, gegebenenfalls N-niederalkyliertes Aminoniederalkyl, Niederalkanoylaminoniederalkyl, Amino, Niederalkylamino, Diniederalkylamino, Niederalkanoylamino, Carboxyl, Niederalkoxycarbonyl, Carbamoyl, N-mono-oder N,N-diniederalkyliertes Carbamoyl, Cyano, Niederalkanoyl, gegebenenfalls durch Niederalkyl, Nitro, Niederalkoxy und/oder Halogen substituiertes Phenyl und/oder Nitro substituiert ist, und $R_3$ Carboxyl, unter physiologischen Bedingungen spaltbares ver estertes Carboxyl oder geschütztes Carboxyl $R_3'$ bedeutet, und Salzen von Verbindungen der Formel I, die eine salzbildende Gruppe aufweisen.

3. Verfahren zur Herstellung von Verbindungen der Formel I gemäss Anspruch 1, worin $R_1$ durch Hydroxy substituiertes Niederalkyl ist, $R_2$ über

ein Ringkohlenstoffatom an den Penem-Rest gebundenes unsubstituiertes oder durch Amino, Niederalkyl und/oder Aminoniederalkyl substituiertes Imidazolyl oder Pyrazolyl, unsubstituiertes oder durch Niederalkyl und/oder Niederalkoxy substituiertes Oxazolyl, unsubstituiertes oder durch Amino, Niederalkylamino, Diniederalkylamino, Niederalkanoylamino, Niederalkyl, Aminoniederalkyl, Hydroxyniederalkyl, Halogenniederalkyl, Carbamoyloxyniederalkyl und/oder Halogen substituiertes Thiazolyl, oder unsubstituiertes oder durch Niederalkyl, substituiertes Isothiazolyl, darstellt, und $R_3$ Carboxyl oder unter physiologischen Bedingungen spaltbares verestertes Carboxyl bedeutet, und Salzen von Verbindungen der Formel I, die eine salzbildende Gruppe aufweisen.

4. Verfahren zur Herstellung von Verbindungen der Formel I gemäss Anspruch 1, worin $R_1$ in $\alpha$-Stellung durch Hydroxy substituiertes Niederalkyl bedeutet, $R_2$ durch Amino und/oder Niederalkyl substituiertes Imidazol-4-yl, durch Amino, Niederalkyl, Hydroxyniederalkyl, Halogenniederalkyl und/oder Aminoniederalkyl substituiertes Thiazol-4-yl oder -5-yl, oder durch Amino substituiertes Isothiazol-4-yl darstellt, und $R_3$ Carboxyl oder unter physiologischen Bedingungen spaltbares verestertes Carboxyl ist, und Salzen von Verbindungen der Formel I, die eine salzbildende Gruppe aufweisen.

5. Verfahren zur Herstellung von Verbindungen der Formel I gemäss Anspruch 1, worin $R_1$ Hydroxymethyl oder 1-Hydroxyäthyl ist, $R_2$ an einem Ringkohlenstoffatom durch Niederalkyl, Amino und/oder Aminoniederalkyl substituiertes Thiazol-4-yl oder Thiazol-5-yl bedeutet und $R_3$ Carboxyl oder unter physiologischen Bedingungen spaltbares verestertes Carboxyl bedeutet, und Salzen von solchen Verbindungen, welche eine salzbildende Gruppe aufweisen.

6. Verfahren zur Herstellung von pharmazeutisch annehmbaren Salzen von Verbindungen der Formel I gemäss Anspruch 1.

7. Verfahren zur Herstellung von unter physiologischen Bedingungen spaltbaren Estern von Verbindungen der Formel I gemäss Anspruch 1.

8. Verfahren zur Herstellung von (5R,6S)-2-(4-Methyl-thiazol-5-yl)-6-hydroxymethyl-2-penem-3-carbonsäure und pharmazeutisch annehmbaren Salzen davon gemäss Anspruch 1.

9. Verfahren zur Herstellung von (5R,6S)-6-[-(1'R)-1'-Hydroxyäthyl]-2-(4-methyl-thiazol-5-yl)-2-penem-3-carbonsaüre und pharmazeutisch annehmbaren Salzen davon gemäss Anspruch 1.

10. Verfahren zur Herstellung von (5R,6S)-2-(2-Amino-4-methyl-thiazol-5-yl)-6-hydroxymethyl-2-penem-3-carbonsäure und pharmazeutisch annehmbaren Salzen davon gemäss Anspruch 1.

11. Verfahren zur Herstellung von (5R,6S)-6-Hydroxymethyl-2-(5-methyl-thiazol-4-yl)-2-penem-3-carbonsäure und pharmazeutisch annehmbaren Salzen davon gemäss Anspruch 1.

12. Verfahren zur Herstellung von (5R,6S)-2-(2-Amino-5-methyl-thiazol-4-yl)-6-hydroxymethyl-2-penem-3-carbonsäure und pharmazeutisch annehmbaren Salzen davon gemäss Anspruch 1.

13. Verfahren zur Herstellung von pharmazeutischen Präparaten, dadurch gekennzeichnet, dass man eine gemäss Anspruch 1 erhältliche Verbindung der Formel I oder ein pharmazeutisch annehmbares Salz davon mit einem pharmazeutisch annehmbaren Trägerstoff mischt.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 86 81 0170

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A- 0 109 362 (CIBA-GEIGY)<br>* Ansprüche * | 1,13,<br>17 | C 07 D 499/00<br>A 61 K 31/43//<br>C 07 F 9/65<br>C 07 F 7/18 |
| | --- | | |
| Y | EP-A- 0 002 210 (MERCK)<br>* Seiten 53,54, Verbindungen 15,16, 18; Seiten 125-126, Verbindungen 15,16,18; Ansprüche * | 1,13,<br>17 | |
| | --- | | |
| Y | EP-A- 0 003 960 (CIBA-GEIGY)<br>* Seiten 15,16; Ansprüche 1-11 * | 1,13,<br>17 | |
| | ------ | | |

RECHERCHIERTE SACHGEBIETE (Int. Cl.4)

C 07 D 499/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-15,17,18

Unvollständig recherchierte Patentansprüche:

Nicht recherchierte Patentansprüche: 16

Grund für die Beschränkung der Recherche:

Verfahren zur chirurgischen oder therapeutischen Behandlung des menschlichen oder tierischen Körpers (siehe Art. 52(4) des Europäischen Patentübereinkommens).

| Recherchenort | Abschlußdatum der Recherche | Prüfer |
|---|---|---|
| Den Haag | 12-06-1986 | CHOULY |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument